# EUROPEAN PATENT APPLICATION

(11) **EP 4 663 633 A1**
(43) Date of publication of application: **17.12.2025**
(21) Application number: 24752922.5
(22) Date of filing: 08.02.2024
(51) Int. Cl.: C07D 401/14, A61K 31/506, A61P 35/00

(54) **COMBINED PHARMACEUTICAL COMPOSITION OF SUBSTITUTED 2-HYDROGEN-PYRAZOLE DERIVATIVE AND USE THEREOF**

(30) Priority: 09.02.2023 CN 202310103670
(71) Applicant: Chia Tai Tianqing Pharmaceutical Group Co., Ltd., Lianyungang, Jiangsu 222062 (CN)
(72) Inventor: JIANG, Wen, Lianyungang, Jiangsu 222062 (CN); FENG, Fan, Lianyungang, Jiangsu 222062 (CN); YU, Ding, Lianyungang, Jiangsu 222062 (CN); WANG, Xunqiang, Lianyungang, Jiangsu 222062 (CN)
(74) Representative: Lorenz Seidler Gossel Part. mbB
(86) International application number: PCT/CN2024/076879
(87) International publication number: WO 2024/165068

(57) **Abstract**

The present disclosure relates to a combined pharmaceutical composition of a substituted 2-hydrogen-pyrazole derivative and the use thereof, and specifically relates to a combined pharmaceutical composition of a compound of formula (I) and the use thereof in the treatment of breast cancer. According to the combined pharmaceutical composition of the present disclosure, the distant relapse-free survival can be remarkably prolonged.

## Description

### CROSS-REFERENCE TO RELATED APPLICATIONS

The present application claims priority and benefit to the Chinese Patent No. 202310103670.5 filed with the National Intellectual Property Administration, PRC on February 09, 2023, which is incorporated herein by reference in its entirety.

### TECHNICAL FIELD

The present disclosure belongs to the technical field of pharmaceutics, relates to a combined pharmaceutical composition of a substituted 2-hydro-pyrazole derivative and use thereof, and particularly relates to a combined pharmaceutical composition of a compound of formula (I) and use thereof for treating breast cancer.

### BACKGROUND

Cyclin-dependent kinase (CDK) 4/6 is a key regulator of the cell cycle, which can trigger the progression of the cell cycle from the growth phase (G1 phase) to the DNA replication phase (S1 phase). In the process of cell proliferation, the complex formed by CDK4/6 and cyclin D can phosphorylate the retinoblastoma protein (Rb). Once the tumor suppressor protein Rb is phosphorylated, it can release transcription factor E2F to which it tightly binds in the unphosphorylated state. E2F activates further transcription to promote the cell cycle to pass the restriction point (R point) and progress from the G1 phase to the S phase. Therefore, the inhibition of CDK4/6 makes it unable to form a cyclin D-CDK4/6 complex, which can block the progression of the cell cycle from the G1 phase to the S phase, thereby achieving the purpose of inhibiting tumor proliferation. WO2016141881 discloses a substituted 2-hydro-pyrazole derivative as a selective CDK4/6 inhibitor, and specifically discloses a compound of formula (I) having the following structure:

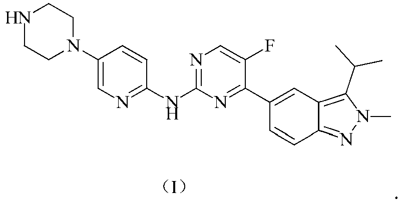

According to guidelines and consensus at home and abroad, an adjuvant endocrine therapy is currently recommended for patients with HR+/HER2- early-stage breast cancer after surgery. For premenopausal patients, a 5-year regimen of ovarian function suppression (OFS) in combination with an aromatase inhibitor (Al) or tamoxifen (TAM), or a 5-year regimen of TAM monotherapy is recommended as an adjuvant endocrine therapy; for postmenopausal patients, a 5-year regimen of aromatase inhibitor (AI) is recommended as an adjuvant endocrine therapy. For patients with high-risk HR+/HER2- early-stage breast cancer, a 5-year regimen of AI + OFS is preferentially recommended. Due to high drug accessibility and good clinical efficacy benefit of endocrine therapeutic agents, the Meta-analysis shows that the 10-year relative recurrence risk of patients with low-risk HR+ and lymph node-negative breast cancer is reduced by 43% after receiving standard adjuvant endocrine therapy, with an absolute benefit reaching 15.6%. However, for HR+ breast cancer patients with high recurrence risk, the currently recommended adjuvant endocrine therapy regimens still need to be optimized, and more precise treatment regimens need to be selected to further reduce the recurrence risk of such patients.

### SUMMARY

In one aspect, the present disclosure provides a combined pharmaceutical composition for use in treating or preventing breast cancer in a patient who has received surgical treatment, comprising a compound of formula (I) or a pharmaceutically acceptable salt thereof and an endocrine therapeutic agent,

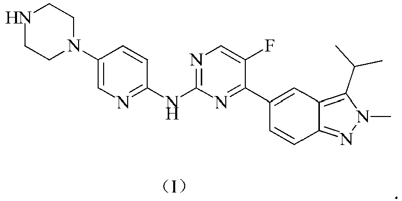

In some embodiments of the present disclosure, the endocrine therapeutic agent is selected from one or two of an aromatase inhibitor, an estrogen receptor antagonist, and a pituitary gonadotropin-releasing hormone analogue. In some embodiments of the present disclosure, the endocrine therapeutic agent is selected from the group consisting of an aromatase inhibitor, an estrogen receptor antagonist, a pituitary gonadotropin-releasing hormone analogue, a combination of an aromatase inhibitor and a pituitary gonadotropin-releasing hormone analogue, and a combination of an estrogen receptor antagonist and a pituitary gonadotropin-releasing hormone analogue.

In another aspect, the present disclosure further provides a kit for use in treating or preventing breast cancer in a patient who has received surgical treatment, comprising: (a) a first pharmaceutical composition comprising the compound of formula (I) or the pharmaceutically acceptable salt thereof described herein; and (b) a second pharmaceutical composition comprising an endocrine therapeutic agent.

In another aspect, the present disclosure provides a combined pharmaceutical composition comprising a compound of formula (I) or a pharmaceutically acceptable salt thereof and an endocrine therapeutic agent, wherein the endocrine therapeutic agent is selected from the group consisting of an estrogen receptor antagonist, a pituitary gonadotropin-releasing hormone analogue, a combination of an aromatase inhibitor and a pituitary gonadotropin-releasing hormone analogue, and a combination of an estrogen receptor antagonist and a pituitary gonadotropin-releasing hormone analogue.

In another aspect, the present disclosure further provides a kit comprising: (a) a first pharmaceutical composition comprising the compound of formula (I) or the pharmaceutically acceptable salt thereof described herein; and (b) a second pharmaceutical composition comprising an endocrine therapeutic agent, wherein the endocrine therapeutic agent is selected from the group consisting of an estrogen receptor antagonist, a pituitary gonadotropin-releasing hormone analogue, a combination of an aromatase inhibitor and the pituitary gonadotropin-releasing hormone analogue, and a combination of an estrogen receptor antagonist and the pituitary gonadotropin-releasing hormone analogue.

In some embodiments of the present disclosure, the aromatase inhibitor is selected from the group consisting of letrozole and anastrozole.

In some embodiments of the present disclosure, the estrogen receptor antagonist is selected from tamoxifen.

In some embodiments of the present disclosure, the pituitary gonadotropin-releasing hormone analogue is selected from goserelin.

In some embodiments of the present disclosure, the endocrine therapeutic agent is selected from one or more, e.g., one or two, of letrozole, anastrozole, tamoxifen, or goserelin.

In some embodiments of the present disclosure, the endocrine therapeutic agent is selected from the group consisting of letrozole, anastrozole, tamoxifen, a combination of letrozole and goserelin, a combination of anastrozole and goserelin, and a combination of tamoxifen and goserelin.

In some embodiments of the present disclosure, the endocrine therapeutic agent is selected from the group consisting of tamoxifen, a combination of letrozole and goserelin, a combination of anastrozole and goserelin, and a combination of tamoxifen and goserelin.

In some embodiments of the present disclosure, the endocrine therapeutic agent is selected from the group consisting of letrozole, anastrozole, and tamoxifen.

In some embodiments of the present disclosure, the endocrine therapeutic agent is selected from tamoxifen.

In another aspect, the present disclosure provides a combined pharmaceutical composition for use in treating or preventing breast cancer in a patient who has received surgical treatment, comprising a compound of formula (I) or a pharmaceutically acceptable salt thereof and an aromatase inhibitor. Alternatively, the present disclosure provides a combined pharmaceutical composition for use in treating or preventing breast cancer in a patient who has received surgical treatment, comprising a combination of a compound of formula (I) or a pharmaceutically acceptable salt thereof, an aromatase inhibitor, and a pituitary gonadotropin-releasing hormone analogue.

In another aspect, the present disclosure provides a combined pharmaceutical composition for use in treating or preventing breast cancer, comprising a compound of formula (I) or a pharmaceutically acceptable salt thereof and tamoxifen.

In another aspect, the present disclosure further provides a method for treating or preventing breast cancer in a patient who has received surgical treatment, comprising administering to an individual in need thereof a therapeutically effective amount of the compound of formula (I) or the pharmaceutically acceptable salt thereof and an endocrine drug, for example, administering to an individual in need thereof a therapeutically effective amount of the combined pharmaceutical composition described herein.

In another aspect, the present disclosure further provides a combination therapy for use in treating an individual with breast cancer who has received surgical treatment, comprising administering to the individual a therapeutically effective amount of the compound of formula (I) or the pharmaceutically acceptable salt thereof alone and administering a therapeutically effective amount of an endocrine drug alone.

In another aspect, the present disclosure further provides a combination therapy for use in treating an individual with breast cancer, comprising administering to the individual a therapeutically effective amount of the compound of formula (I) or the pharmaceutically acceptable salt thereof alone and administering a therapeutically effective amount of an endocrine therapeutic agent alone, wherein the endocrine therapeutic agent is selected from the group consisting of an estrogen receptor antagonist, a pituitary gonadotropin-releasing hormone analogue, a combination of an aromatase inhibitor and a pituitary gonadotropin-releasing hormone analogue, and a combination of an estrogen receptor antagonist and a pituitary gonadotropin-releasing hormone analogue.

In another aspect, the present disclosure further provides use of the compound of formula (I) or the pharmaceutically acceptable salt thereof in combination with an endocrine drug for preparing a medicament for treating or preventing breast cancer in a patient who has received surgical treatment, for example, use of the combined pharmaceutical composition described herein for preparing a medicament for treating or preventing breast cancer in a patient who has received surgical treatment. In some embodiments of the present disclosure, the combined pharmaceutical composition is the combined pharmaceutical composition described herein.

In another aspect, the present disclosure further provides use of the compound of formula (I) or the pharmaceutically acceptable salt thereof in combination with an endocrine drug for treating or preventing breast cancer in a patient who has received surgical treatment, for example, use of the combined pharmaceutical composition described herein for treating or preventing breast cancer in a patient who has received surgical treatment.

In some embodiments of the present disclosure, the compound of formula (I) or the pharmaceutically acceptable salt thereof and the endocrine therapeutic agent in the combined pharmaceutical composition are packaged in the same kit, and the kit further comprises an instruction for use of the compound of formula (I) or the pharmaceutically acceptable salt thereof in combination with the endocrine therapeutic agent for treating or preventing breast cancer in a patient who has received surgical treatment.

In some embodiments of the present disclosure, the compound of formula (I) or the pharmaceutically acceptable salt thereof and the endocrine therapeutic agent in the combined pharmaceutical composition are packaged separately in their respective kits, and the kits further comprise instructions for use of the compound of formula (I) or the pharmaceutically acceptable salt thereof in combination with the endocrine therapeutic agent for treating or preventing breast cancer in a patient who has received surgical treatment.

In some embodiments of the present disclosure, the compound of formula (I) or the pharmaceutically acceptable salt thereof and the endocrine therapeutic agent in the combined pharmaceutical composition are each in the form of a pharmaceutical composition and can be administered simultaneously, sequentially, or at intervals.

In some embodiments of the present disclosure, the combined pharmaceutical composition comprises a pharmaceutical composition of the compound of formula (I) or the pharmaceutically acceptable salt thereof, and a pharmaceutical composition of an endocrine therapeutic agent.

In some embodiments of the present disclosure, the pharmaceutical composition of the compound of formula (I) or the pharmaceutically acceptable salt thereof and the pharmaceutical composition of the endocrine therapeutic agent in the combined pharmaceutical composition are packaged in the same kit, and the kit further comprises an instruction for use of the pharmaceutical composition of the compound of formula (I) or the pharmaceutically acceptable salt thereof in combination with the pharmaceutical composition of the endocrine therapeutic agent for treating or preventing breast cancer in a patient who has received surgical treatment.

In some embodiments of the present disclosure, the pharmaceutical composition of the compound of formula (I) or the pharmaceutically acceptable salt thereof and the pharmaceutical composition of the endocrine therapeutic agent in the combined pharmaceutical composition are packaged separately in their respective kits, and the kits further comprise instructions for use of the pharmaceutical composition of the compound of formula (I) or the pharmaceutically acceptable salt thereof in combination with the pharmaceutical composition of the endocrine therapeutic agent for treating or preventing breast cancer in a patient who has received surgical treatment.

In some embodiments of the present disclosure, in the combined pharmaceutical composition, the pharmaceutically acceptable salt of the compound of formula (I) is a maleate, e.g., a monomaleate of the compound of formula (I).

In some embodiments of the present disclosure, the aromatase inhibitor is selected from the group consisting of letrozole and anastrozole.

In some embodiments of the present disclosure, the combined pharmaceutical composition comprises 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I), or a pharmaceutical composition thereof. Alternatively, the combined pharmaceutical composition is in the form of a single-administration formulation, and the combined pharmaceutical composition comprises 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I), or a pharmaceutical composition thereof.

In some embodiments of the present disclosure, the combined pharmaceutical composition comprises 20 mg, 40 mg, 60 mg, 80 mg, 100 mg, 120 mg, 150 mg, 180 mg, or 240 mg of the compound of formula (I) or the pharmaceutically acceptable salt thereof based on the compound of formula (I), or a pharmaceutical composition thereof.

In some embodiments of the present disclosure, the combined pharmaceutical composition comprises 120 mg, 150 mg, or 180 mg of the compound of formula (I) or the pharmaceutically acceptable salt thereof based on the compound of formula (I), or a pharmaceutical composition thereof. Alternatively, the combined pharmaceutical composition is in the form of a single-administration formulation, and the combined pharmaceutical composition comprises 120 mg, 150 mg, or 180 mg of the compound of formula (I) or the pharmaceutically acceptable salt thereof based on the compound of formula (I), or a pharmaceutical composition thereof.

In some embodiments of the present disclosure, in the combined pharmaceutical composition, the pharmaceutical composition of the compound of formula (I) or the pharmaceutically acceptable salt thereof is in a single-dose form or a multi-dose form; in some embodiments of the present disclosure, in the combined pharmaceutical composition, the pharmaceutical composition of the compound of formula (I) or the pharmaceutically acceptable salt thereof is in a multi-dose form.

In some embodiments of the present disclosure, in the combined pharmaceutical composition, the content of the compound of formula (I) or the pharmaceutically acceptable salt thereof is a daily dose or a one-day dose.

In some embodiments of the present disclosure, in the combined pharmaceutical composition, the content of the compound of formula (I) or the pharmaceutically acceptable salt thereof is a once-daily dose or a once-a-day dose. In some embodiments of the present disclosure, in the combined pharmaceutical composition, the content of the compound of formula (I) or the pharmaceutically acceptable salt thereof is a once-daily dose or a once-a-day dose, with each dose being a single dose or multiple doses, preferably multiple doses.

In some embodiments of the present disclosure, the combined pharmaceutical composition comprises a single dose of 50 mg, 60 mg, 120 mg, 150 mg, or 180 mg of a pharmaceutical composition of the compound of formula (I) or the pharmaceutically acceptable salt thereof based on the compound of formula (I).

In some embodiments of the present disclosure, the combined pharmaceutical composition comprises a single dose of 50 mg or 60 mg of a pharmaceutical composition of the compound of formula (I) or the pharmaceutically acceptable salt thereof based on the compound of formula (I).

In some embodiments of the present disclosure, the combined pharmaceutical composition comprises a single dose of 60 mg of a pharmaceutical composition of the compound of formula (I) or the pharmaceutically acceptable salt thereof based on the compound of formula (I).

In some embodiments of the present disclosure, the combined pharmaceutical composition comprises the compound of formula (I) or the pharmaceutically acceptable salt thereof and the endocrine therapeutic agent in a mass ratio of 1:1 to 200:1, preferably 10:1 to 200:1, 6:1 to 180:1, 6:1 to 90:1, 6:1 to 9:1, 48:1 to 72:1, 120:1 to 180:1, or any ratio within the range described above, wherein the amount of the compound of formula (I) or the pharmaceutically acceptable salt thereof is based on the mass of the compound of formula (I), and the amount of the endocrine therapeutic agent is based on the mass of an active ingredient therein.

In some embodiments of the present disclosure, the combined pharmaceutical composition comprises the compound of formula (I) or the pharmaceutically acceptable salt thereof and the aromatase inhibitor in a mass ratio of 1:1 to 200:1, preferably 10:1 to 200:1, 20:1 to 200:1, 40:1 to 180:1, 48:1 to 72:1, 120:1 to 180:1, or any ratio within the range described above, wherein the amount of the compound of formula (I) or the pharmaceutically acceptable salt thereof is based on the mass of the compound of formula (I), and the amount of the aromatase inhibitor is based on the mass of an active ingredient therein.

In some embodiments of the present disclosure, the combined pharmaceutical composition comprises the compound of formula (I) or the pharmaceutically acceptable salt thereof and the estrogen receptor antagonist in a mass ratio of 1:1 to 200:1, preferably 1.5:1 to 180:1, 6:1 to 180:1, 6:1 to 90:1, or any ratio within the range described above, wherein the amount of the compound of formula (I) or the pharmaceutically acceptable salt thereof is based on the mass of the compound of formula (I), and the amount of the estrogen receptor antagonist is based on the mass of an active ingredient therein.

In some embodiments of the present disclosure, the pharmaceutical composition of the compound of formula (I) or the pharmaceutically acceptable salt thereof comprised in the combined pharmaceutical composition is packaged in a kit, and the kit further comprises an instruction for use of the compound of formula (I) or the pharmaceutically acceptable salt thereof for treating or preventing breast cancer in a patient who has received surgical treatment.

### Combined Pharmaceutical Composition of Compound of Formula (I) or Pharmaceutically Acceptable Salt Thereof and Letrozole

In some embodiments of the present disclosure, the present disclosure provides a combined pharmaceutical composition for use in treating or preventing breast cancer in a patient who has received surgical treatment, comprising the compound of formula (I) or the pharmaceutically acceptable salt thereof and letrozole.

In some embodiments of the present disclosure, the combined pharmaceutical composition comprises 2.5-12.5 mg, 2.5-10 mg, 2.5-7.5 mg, or 2.5-5 mg of letrozole or a pharmaceutical composition thereof. Alternatively, the combined pharmaceutical composition is in the form of a single-administration formulation, and the combined pharmaceutical composition comprises 2.5-12.5 mg, 2.5-10 mg, 2.5-7.5 mg, or 2.5-5 mg of letrozole or a pharmaceutical composition thereof.

In some embodiments of the present disclosure, the combined pharmaceutical composition comprises 2.5 mg, 5 mg, 7.5 mg, or 10 mg of letrozole or a pharmaceutical composition thereof. In some embodiments of the present disclosure, the combined pharmaceutical composition comprises 2.5 mg of letrozole or a pharmaceutical composition thereof.

In some embodiments of the present disclosure, in the combined pharmaceutical composition, the pharmaceutical composition of letrozole is in a single-dose form or a multi-dose form. In some embodiments of the present disclosure, in the combined pharmaceutical composition, the pharmaceutical composition of letrozole is in a single-dose form. In some embodiments of the present disclosure, in the combined pharmaceutical composition, the content of letrozole is a daily dose or a one-day dose. In some embodiments of the present disclosure, in the combined pharmaceutical composition, the content of letrozole is a once-daily dose or a once-a-day dose. In some embodiments of the present disclosure, in the combined pharmaceutical composition, the content of letrozole is a once-daily dose or a once-a-day dose, with each dose being a single dose or multiple doses, preferably a single dose.

In some embodiments of the present disclosure, the combined pharmaceutical composition comprises a single dose of 2.5 mg of a pharmaceutical composition of letrozole.

In some embodiments of the present disclosure, the pharmaceutical composition of letrozole comprised in the combined pharmaceutical composition is packaged in a kit, and the kit further comprises an instruction for use of letrozole for treating or preventing breast cancer in a patient who has received surgical treatment.

In some embodiments of the present disclosure, the pharmaceutical composition of letrozole comprised in the combined pharmaceutical composition is packaged in a kit, the kit further comprises an instruction for use of letrozole for treating or preventing breast cancer in a patient who has received surgical treatment, and the instruction may be an instruction in a package insert of a commercially available letrozole kit.

In some embodiments of the present disclosure, the combined pharmaceutical composition comprises 20-240 mg of the compound of formula (I) or the pharmaceutically acceptable salt thereof based on the compound of formula (I), and 2.5-12.5 mg of letrozole.

In some embodiments of the present disclosure, the combined pharmaceutical composition comprises 120-180 mg of the compound of formula (I) or the pharmaceutically acceptable salt thereof based on the compound of formula (I), and 2.5-5 mg of letrozole.

In some embodiments of the present disclosure, the combined pharmaceutical composition comprises a daily dose of 20 mg, 40 mg, 60 mg, 80 mg, 100 mg, 120 mg, 150 mg, 180 mg, or 240 mg of the compound of formula (I) or the pharmaceutically acceptable salt thereof based on the compound of formula (I), and a daily dose of 2.5 mg of letrozole.

In some embodiments of the present disclosure, the combined pharmaceutical composition comprises a daily dose of 120 mg, 150 mg, or 180 mg of the compound of formula (I) or the pharmaceutically acceptable salt thereof based on the compound of formula (I), and a daily dose of 2.5 mg of letrozole.

In some embodiments of the present disclosure, the combined pharmaceutical composition comprises a single dose of 50 mg or 60 mg of a pharmaceutical composition of the compound of formula (I) or the pharmaceutically acceptable salt thereof based on the compound of formula (I), and a single dose of 2.5 mg of a pharmaceutical composition of letrozole.

In some embodiments of the present disclosure, the combined pharmaceutical composition comprises a single dose of 60 mg of a pharmaceutical composition of the compound of formula (I) or the pharmaceutically acceptable salt thereof based on the compound of formula (I), and a single dose of 2.5 mg of a pharmaceutical composition of letrozole.

In some embodiments of the present disclosure, the combined pharmaceutical composition comprises the compound of formula (I) or the pharmaceutically acceptable salt thereof or the pharmaceutical composition thereof and letrozole or the pharmaceutical composition thereof in a mass ratio of 1:1 to 200:1, preferably 10:1 to 200:1, 20:1 to 150:1, 24:1 to 144:1, 24:1 to 72:1, 48:1 to 72:1, or any ratio within the range described above, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole.

In some embodiments of the present disclosure, the combined pharmaceutical composition is a formulation suitable for administration in a single treatment cycle (e.g., 28 days), and the formulation comprises: a total amount of 1680-5040 mg (e.g., 3360-5040 mg, preferably 3360 mg, 4200 mg, or 5040 mg) of the compound of formula (I) or the pharmaceutically acceptable salt thereof or a pharmaceutical composition thereof; and a total amount of 70-140 mg (e.g., 70 mg) of letrozole or a pharmaceutical composition thereof, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole. In some preferred embodiments, the compound of formula (I) or the pharmaceutically acceptable salt thereof or the pharmaceutical composition thereof is packaged in a single aliquot or multiple aliquots (e.g., 2, 4, 7, 14, 28 or more aliquots). In some preferred embodiments, letrozole or the pharmaceutical composition thereof is packaged in a single aliquot or multiple aliquots (e.g., 2, 4, 7, 14, 28 or more aliquots). In some embodiments, the compound of formula (I) or the pharmaceutically acceptable salt thereof or the pharmaceutical composition thereof and letrozole or the pharmaceutical composition thereof are packaged separately or together.

In some embodiments of the present disclosure, the combined pharmaceutical composition is a formulation suitable for administration in a single treatment cycle (e.g., 28 days), and the formulation comprises the compound of formula (I) or the pharmaceutically acceptable salt thereof or a pharmaceutical composition thereof and letrozole or a pharmaceutical composition thereof in a mass ratio of 12:1 to 72:1, e.g., 24:1 to 72:1, 48:1 to 72:1, 48:1, 60:1, 72:1, or any ratio within the range described above, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole.

In some embodiments of the present disclosure, in the combined pharmaceutical composition, the compound of formula (I) or the pharmaceutically acceptable salt thereof and letrozole may be each in the form of a pharmaceutical composition or be in the form of a pharmaceutical composition together.

In another aspect, the present disclosure further provides a kit for use in treating or preventing breast cancer in a patient who has received surgical treatment, comprising: (a) a first pharmaceutical composition comprising the compound of formula (I) or the pharmaceutically acceptable salt thereof described herein; and (b) a second pharmaceutical composition comprising letrozole.

In another aspect, the present disclosure further provides a kit comprising a pharmaceutical composition for use in treating or preventing breast cancer in a patient who has received surgical treatment, comprising: (a) a first pharmaceutical composition comprising the compound of formula (I) or the pharmaceutically acceptable salt thereof described herein; and (b) a second pharmaceutical composition comprising letrozole.

In another aspect, the present disclosure further provides a method for treating or preventing breast cancer in a patient who has received surgical treatment, comprising administering to an individual in need thereof a therapeutically effective amount of the compound of formula (I) or the pharmaceutically acceptable salt thereof and letrozole, for example, administering to an individual in need thereof a therapeutically effective amount of the combined pharmaceutical composition described herein.

In another aspect, the present disclosure further provides a combination therapy for use in treating an individual with breast cancer who has received surgical treatment, comprising administering to the individual a therapeutically effective amount of the compound of formula (I) or the pharmaceutically acceptable salt thereof alone and administering a therapeutically effective amount of letrozole alone.

In another aspect, the present disclosure further provides use of the compound of formula (I) or the pharmaceutically acceptable salt thereof in combination with letrozole for preparing a medicament for treating or preventing breast cancer in a patient who has received surgical treatment, for example, use of the combined pharmaceutical composition described herein for preparing a medicament for treating or preventing breast cancer in a patient who has received surgical treatment. In some embodiments of the present disclosure, the combined pharmaceutical composition is the combined pharmaceutical composition described herein.

In another aspect, the present disclosure further provides use of the compound of formula (I) or the pharmaceutically acceptable salt thereof in combination with letrozole for treating or preventing breast cancer in a patient who has received surgical treatment, for example, use of the combined pharmaceutical composition described above herein for treating or preventing breast cancer in a patient who has received surgical treatment.

In some embodiments of the present disclosure, in the kit, the method, the combination therapy, or the use, the definitions of the compound of formula (I) or the pharmaceutically acceptable salt thereof and letrozole are the same as those of the compound of formula (I) or the pharmaceutically acceptable salt thereof and letrozole in the combined pharmaceutical composition described herein, for example, in terms of content, dose, form of presence, form of packaging, or the like.

In some embodiments of the present disclosure, in the method, the combination therapy, or the use, the compound of formula (I) or the pharmaceutically acceptable salt thereof and letrozole are each in the form of a pharmaceutical composition and can be administered simultaneously, separately, concurrently, sequentially, or at intervals.

In some embodiments of the present disclosure, in the method, the combination therapy, or the use, the compound of formula (I) or the pharmaceutically acceptable salt thereof and letrozole each have the same or different treatment cycles. In some specific embodiments of the present disclosure, in the method, the combination therapy, or the use, the compound of formula (I) or the pharmaceutically acceptable salt thereof and letrozole have the same treatment cycle, e.g., a 1-week, 2-week, 3-week or 4-week treatment cycle.

In some embodiments of the present disclosure, in the method, the combination therapy, or the use, the content of the compound of formula (I) or the pharmaceutically acceptable salt thereof in the combined pharmaceutical composition is a daily dose, which is administered by administering the compound of formula (I) or the pharmaceutically acceptable salt thereof once daily.

In some embodiments of the present disclosure, in the method, the combination therapy, or the use, the content of the compound of formula (I) or the pharmaceutically acceptable salt thereof in the combined pharmaceutical composition is a daily dose, wherein the compound of formula (I) or the pharmaceutically acceptable salt thereof is administered in a single dose or multiple doses, typically in multiple doses; further, the compound of formula (I) or the pharmaceutically acceptable salt thereof is administered once daily.

In some embodiments of the present disclosure, in the method, the combination therapy, or the use, the compound of formula (I) or the pharmaceutically acceptable salt thereof is administered in a daily dose of 120 mg, in a daily dose of 150 mg, or in a daily dose of 180 mg.

In some embodiments of the present disclosure, in the method, the combination therapy, or the use, the compound of formula (I) or the pharmaceutically acceptable salt thereof in the combined pharmaceutical composition is administered in multiple doses consisting of single doses of 50 mg or 60 mg of the pharmaceutical composition of the compound of formula (I) or the pharmaceutically acceptable salt thereof, preferably in multiple doses consisting of single doses of 60 mg of the pharmaceutical composition of the compound of formula (I) or the pharmaceutically acceptable salt thereof. In some embodiments of the present disclosure, in the method, the combination therapy, or the use, the compound of formula (I) or the pharmaceutically acceptable salt thereof is administered on a regimen of consecutive daily administration.

In some embodiments of the present disclosure, in the method, the combination therapy, or the use, the content of the compound of formula (I) or the pharmaceutically acceptable salt thereof in the combined pharmaceutical composition is a dose per treatment cycle, which is administered by administering the compound of formula (I) or the pharmaceutically acceptable salt thereof daily.

In some embodiments of the present disclosure, in the method, the combination therapy, or the use, the content of letrozole in the combined pharmaceutical composition is a daily dose, which is administered by administering letrozole once daily.

In some embodiments of the present disclosure, in the method, the combination therapy, or the use, the content of letrozole in the combined pharmaceutical composition is a daily dose, wherein letrozole is administered in a single dose or multiple doses, typically in a single dose; further, letrozole is administered once daily.

In some embodiments of the present disclosure, in the method, the combination therapy, or the use, letrozole is administered in a daily dose of 2.5 mg.

In some embodiments of the present disclosure, in the method, the combination therapy, or the use, letrozole in the combined pharmaceutical composition is administered in a single dose of 2.5 mg of the pharmaceutical composition of letrozole. In some embodiments of the present disclosure, in the method, the combination therapy, or the use, letrozole is administered on a regimen of consecutive daily administration.

In some embodiments of the present disclosure, in the method, the combination therapy, or the use, the content of letrozole in the combined pharmaceutical composition is a dose per treatment cycle, which is administered by administering letrozole daily.

In some embodiments of the present disclosure, in the method, the combination therapy, or the use, 28 days are counted as one treatment cycle, and the compound of formula (I) or the pharmaceutically acceptable salt thereof and letrozole are administered daily on days 1-28 in each treatment cycle.

In some specific embodiments of the present disclosure, in the method, the combination therapy, or the use, 28 days are counted as one treatment cycle, and the compound of formula (I) or the pharmaceutically acceptable salt thereof and letrozole are administered once daily on days 1-28 in each treatment cycle.

In some embodiments of the present disclosure, in the method, the combination therapy, or the use, 28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 3360-5040 mg. In some embodiments of the present disclosure, the total dose of the pharmaceutical composition comprising the compound of formula (I) or the pharmaceutically acceptable salt thereof is selected from the group consisting of 3360 mg, 4200 mg, 5040 mg, and a range formed by any two of the aforementioned values. In some embodiments of the present disclosure, the total dose of the pharmaceutical composition comprising the compound of formula (I) or the pharmaceutically acceptable salt thereof is preferably 5040 mg.

In some embodiments of the present disclosure, in the method, the combination therapy, or the use, 28 days are counted as one treatment cycle, the administration is performed once daily for 28 consecutive days, and a total dose of the pharmaceutical composition comprising letrozole administered per treatment cycle is 70-210 mg. In some embodiments of the present disclosure, the total dose of the pharmaceutical composition comprising letrozole is selected from the group consisting of 70 mg, 140 mg, 210 mg, and a range formed by any two of the aforementioned values. In some embodiments of the present disclosure, the total dose of the pharmaceutical composition comprising letrozole is preferably 70 mg.

In some embodiments of the present disclosure, in the method, the combination therapy, or the use, 28 days are counted as one treatment cycle, the administration is performed once daily for 28 consecutive days, and a total dose of 5040 mg of the pharmaceutical composition comprising the compound of formula (I) or the pharmaceutically acceptable salt thereof and a total dose of 70 mg of the pharmaceutical composition comprising letrozole are administered per treatment cycle.

In embodiments of the present disclosure, the treatment cycle described above is repeated as long as the disease is still under control and the administration regimen is clinically tolerable.

In some embodiments of the present disclosure, letrozole is prepared as a single dose or multiple doses suitable for consecutive daily administration of 2.5-7.5 mg or 2.5-5 mg of letrozole to a patient; and the pharmaceutical composition comprising the compound of formula (I) or the pharmaceutically acceptable salt thereof is prepared as a single dose or multiple doses suitable for consecutive daily administration of 120 mg, 150 mg, and/or 180 mg based on the compound of formula (I) to a patient.

In some embodiments of the present disclosure, letrozole is prepared as a single dose suitable for consecutive daily administration of 2.5 mg of letrozole to a patient; and the pharmaceutical composition comprising the compound of formula (I) or the pharmaceutically acceptable salt thereof is prepared as multiple doses suitable for consecutive daily administration of 180 mg based on the compound of formula (I) to a patient.

### Combined Pharmaceutical Composition of Compound of Formula (I) or Pharmaceutically Acceptable Salt Thereof and Anastrozole

In some embodiments of the present disclosure, the present disclosure provides a combined pharmaceutical composition for use in treating or preventing breast cancer in a patient who has received surgical treatment, comprising the compound of formula (I) or the pharmaceutically acceptable salt thereof and anastrozole.

In some embodiments of the present disclosure, the combined pharmaceutical composition comprises 1-5 mg, 1-4 mg, 1-3 mg, or 1-2 mg of anastrozole or a pharmaceutical composition thereof. Alternatively, the combined pharmaceutical composition is in the form of a single-administration formulation, and the combined pharmaceutical composition comprises 1-5 mg, 1-4 mg, 1-3 mg, or 1-2 mg of anastrozole or a pharmaceutical composition thereof.

In some embodiments of the present disclosure, the combined pharmaceutical composition comprises 1 mg, 2 mg, 3 mg, or 4 mg of anastrozole or a pharmaceutical composition thereof. In some embodiments of the present disclosure, the combined pharmaceutical composition comprises 1 mg of anastrozole or a pharmaceutical composition thereof.

In some embodiments of the present disclosure, in the combined pharmaceutical composition, the pharmaceutical composition of anastrozole is in a single-dose form or a multi-dose form. In some embodiments of the present disclosure, in the combined pharmaceutical composition, the pharmaceutical composition of anastrozole is in a single-dose form. In some embodiments of the present disclosure, in the combined pharmaceutical composition, the content of anastrozole is a daily dose or a one-day dose. In some embodiments of the present disclosure, in the combined pharmaceutical composition, the content of anastrozole is a once-daily dose or a once-a-day dose. In some embodiments of the present disclosure, in the combined pharmaceutical composition, the content of anastrozole is a once-daily dose or a once-a-day dose, with each dose being a single dose or multiple doses, preferably a single dose.

In some embodiments of the present disclosure, the combined pharmaceutical composition comprises a single dose of 1 mg of a pharmaceutical composition of anastrozole.

In some embodiments of the present disclosure, the pharmaceutical composition of anastrozole comprised in the combined pharmaceutical composition is packaged in a kit, and the kit further comprises an instruction for use of anastrozole for treating or preventing breast cancer in a patient who has received surgical treatment.

In some embodiments of the present disclosure, the pharmaceutical composition of anastrozole comprised in the combined pharmaceutical composition is packaged in a kit, the kit further comprises an instruction for use of anastrozole for treating or preventing breast cancer in a patient who has received surgical treatment, and the instruction may be an instruction in a package insert of a commercially available anastrozole kit.

In some embodiments of the present disclosure, the combined pharmaceutical composition comprises 20-240 mg of the compound of formula (I) or the pharmaceutically acceptable salt thereof based on the compound of formula (I), and 1-5 mg of anastrozole.

In some embodiments of the present disclosure, the combined pharmaceutical composition comprises 120-180 mg of the compound of formula (I) or the pharmaceutically acceptable salt thereof based on the compound of formula (I), and 1-2 mg of anastrozole.

In some embodiments of the present disclosure, the combined pharmaceutical composition comprises a daily dose of 20 mg, 40 mg, 60 mg, 80 mg, 100 mg, 120 mg, 150 mg, 180 mg, or 240 mg of the compound of formula (I) or the pharmaceutically acceptable salt thereof based on the compound of formula (I), and a daily dose of 1 mg of anastrozole.

In some embodiments of the present disclosure, the combined pharmaceutical composition comprises 120 mg, 150 mg, or 180 mg of a daily dose of the compound of formula (I) or the pharmaceutically acceptable salt thereof based on the compound of formula (I), and a daily dose of 1 mg of anastrozole.

In some embodiments of the present disclosure, the combined pharmaceutical composition comprises a single dose of 50 mg or 60 mg of a pharmaceutical composition of the compound of formula (I) or the pharmaceutically acceptable salt thereof based on the compound of formula (I), and a single dose of 1 mg of a pharmaceutical composition of anastrozole.

In some embodiments of the present disclosure, the combined pharmaceutical composition comprises a single dose of 60 mg of a pharmaceutical composition of the compound of formula (I) or the pharmaceutically acceptable salt thereof based on the compound of formula (I), and a single dose of 1 mg of a pharmaceutical composition of anastrozole.

In some embodiments of the present disclosure, the combined pharmaceutical composition comprises the compound of formula (I) or the pharmaceutically acceptable salt thereof or the pharmaceutical composition thereof and anastrozole or the pharmaceutical composition thereof in a mass ratio of 5:1 to 200:1, preferably 6:1 to 180:1, 10:1 to 180:1, 50:1 to 180:1, 60:1 to 180:1, 120:1 to 180:1, 150:1 to 180:1, or any ratio within the range described above, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

In some embodiments of the present disclosure, the combined pharmaceutical composition is a formulation suitable for administration in a single treatment cycle (e.g., 28 days), and the formulation comprises: a total amount of 1680-5040 mg (e.g., 3360-5040 mg, preferably 3360 mg, 4200 mg, or 5040 mg) of the compound of formula (I) or the pharmaceutically acceptable salt thereof or a pharmaceutical composition thereof; and a total amount of 28-140 mg (e.g., 28-56 mg, preferably 28 mg) of anastrozole or a pharmaceutical composition thereof, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole. In some preferred embodiments, the compound of formula (I) or the pharmaceutically acceptable salt thereof or the pharmaceutical composition thereof is packaged in a single aliquot or multiple aliquots (e.g., 2, 4, 7, 14, 28 or more aliquots). In some preferred embodiments, anastrozole or the pharmaceutical composition thereof is packaged in a single aliquot or multiple aliquots (e.g., 2, 4, 7, 14, 28 or more aliquots). In some embodiments, the compound of formula (I) or the pharmaceutically acceptable salt thereof or the pharmaceutical composition thereof and anastrozole or the pharmaceutical composition thereof are packaged separately or together.

In some embodiments of the present disclosure, the combined pharmaceutical composition is a formulation suitable for administration in a single treatment cycle (e.g., 28 days), and the formulation comprises the compound of formula (I) or the pharmaceutically acceptable salt thereof or a pharmaceutical composition thereof and anastrozole or a pharmaceutical composition thereof in a mass ratio of 12:1 to 180:1, e.g., 24:1 to 180:1, 30:1 to 180:1, 60:1 to 180:1, 120:1 to 180:1, 150:1 to 180:1, 180:1, or any ratio within the range described above, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

In some embodiments of the present disclosure, in the combined pharmaceutical composition, the compound of formula (I) or the pharmaceutically acceptable salt thereof and anastrozole may be each in the form of a pharmaceutical composition or be in the form of a pharmaceutical composition together.

In another aspect, the present disclosure further provides a kit for use in treating or preventing breast cancer in a patient who has received surgical treatment, comprising: (a) a first pharmaceutical composition comprising the compound of formula (I) or the pharmaceutically acceptable salt thereof described herein; and (b) a second pharmaceutical composition comprising anastrozole.

In another aspect, the present disclosure further provides a kit comprising a pharmaceutical composition for use in treating or preventing breast cancer in a patient who has received surgical treatment, comprising: (a) a first pharmaceutical composition comprising the compound of formula (I) or the pharmaceutically acceptable salt thereof described herein; and (b) a second pharmaceutical composition comprising anastrozole.

In another aspect, the present disclosure further provides a method for treating or preventing breast cancer in a patient who has received surgical treatment, comprising administering to an individual in need thereof a therapeutically effective amount of the compound of formula (I) or the pharmaceutically acceptable salt thereof and anastrozole, for example, administering to an individual in need thereof a therapeutically effective amount of the combined pharmaceutical composition described herein.

In another aspect, the present disclosure further provides a combination therapy for use in treating an individual with breast cancer who has received surgical treatment, comprising administering to the individual a therapeutically effective amount of the compound of formula (I) or the pharmaceutically acceptable salt thereof alone and administering a therapeutically effective amount of anastrozole alone.

In another aspect, the present disclosure further provides use of the compound of formula (I) or the pharmaceutically acceptable salt thereof in combination with anastrozole for preparing a medicament for treating or preventing breast cancer in a patient who has received surgical treatment, for example, use of the combined pharmaceutical composition described herein for preparing a medicament for treating or preventing breast cancer in a patient who has received surgical treatment. In some embodiments of the present disclosure, the combined pharmaceutical composition is the combined pharmaceutical composition described herein.

In another aspect, the present disclosure further provides use of the compound of formula (I) or the pharmaceutically acceptable salt thereof in combination with anastrozole for treating or preventing breast cancer in a patient who has received surgical treatment, for example, use of the combined pharmaceutical composition described herein for treating or preventing breast cancer in a patient who has received surgical treatment.

In some embodiments of the present disclosure, in the kit, the method, the combination therapy, or the use, the definitions of the compound of formula (I) or the pharmaceutically acceptable salt thereof and anastrozole are the same as those of the compound of formula (I) or the pharmaceutically acceptable salt thereof and anastrozole in the combined pharmaceutical composition described herein, for example, in terms of content, dose, form of presence, form of packaging, or the like.

In some embodiments of the present disclosure, in the method, the combination therapy, or the use, the compound of formula (I) or the pharmaceutically acceptable salt thereof and anastrozole are each in the form of a pharmaceutical composition and can be administered simultaneously, separately, concurrently, sequentially, or at intervals.

In some embodiments of the present disclosure, in the method, the combination therapy, or the use, the compound of formula (I) or the pharmaceutically acceptable salt thereof and anastrozole each have the same or different treatment cycles. In some specific embodiments of the present disclosure, in the method, the combination therapy, or the use, the compound of formula (I) or the pharmaceutically acceptable salt thereof and anastrozole have the same treatment cycle, e.g., a 1-week, 2-week, 3-week or 4-week treatment cycle.

In some embodiments of the present disclosure, in the method, the combination therapy, or the use, the content of the compound of formula (I) or the pharmaceutically acceptable salt thereof in the combined pharmaceutical composition is a daily dose, which is administered by administering the compound of formula (I) or the pharmaceutically acceptable salt thereof once daily.

In some embodiments of the present disclosure, in the method, the combination therapy, or the use, the content of the compound of formula (I) or the pharmaceutically acceptable salt thereof in the combined pharmaceutical composition is a daily dose, wherein the compound of formula (I) or the pharmaceutically acceptable salt thereof is administered in a single dose or multiple doses, typically in multiple doses; further, the compound of formula (I) or the pharmaceutically acceptable salt thereof is administered once daily.

In some embodiments of the present disclosure, in the method, the combination therapy, or the use, the compound of formula (I) or the pharmaceutically acceptable salt thereof is administered in a daily dose of 120 mg, in a daily dose of 150 mg, or in a daily dose of 180 mg.

In some embodiments of the present disclosure, in the method, the combination therapy, or the use, the compound of formula (I) or the pharmaceutically acceptable salt thereof in the combined pharmaceutical composition is administered in multiple doses consisting of single doses of 50 mg or 60 mg of the pharmaceutical composition of the compound of formula (I) or the pharmaceutically acceptable salt thereof, preferably in multiple doses consisting of single doses of 60 mg of the pharmaceutical composition of the compound of formula (I) or the pharmaceutically acceptable salt thereof. In some embodiments of the present disclosure, in the method, the combination therapy, or the use, the compound of formula (I) or the pharmaceutically acceptable salt thereof is administered on a regimen of consecutive daily administration.

In some embodiments of the present disclosure, in the method, the combination therapy, or the use, the content of the compound of formula (I) or the pharmaceutically acceptable salt thereof in the combined pharmaceutical composition is a dose per treatment cycle, which is administered by administering the compound of formula (I) or the pharmaceutically acceptable salt thereof daily.

In some embodiments of the present disclosure, in the method, the combination therapy, or the use, the content of anastrozole in the combined pharmaceutical composition is a daily dose, which is administered by administering anastrozole once daily.

In some embodiments of the present disclosure, in the method, the combination therapy, or the use, the content of anastrozole in the combined pharmaceutical composition is a daily dose, wherein anastrozole is administered in a single dose or in multiple doses, typically in a single dose; further, anastrozole is administered once daily.

In some embodiments of the present disclosure, in the method, the combination therapy, or the use, anastrozole is administered in a daily dose of 1 mg.

In some embodiments of the present disclosure, in the method, the combination therapy, or the use, anastrozole in the combined pharmaceutical composition is administered in a single dose of 1 mg of the pharmaceutical composition of anastrozole. In some embodiments of the present disclosure, in the method, the combination therapy, or the use, anastrozole is administered on a regimen of consecutive daily administration.

In some embodiments of the present disclosure, in the method, the combination therapy, or the use, the content of anastrozole in the combined pharmaceutical composition is a dose per treatment cycle, which is administered by administering anastrozole daily.

In some embodiments of the present disclosure, in the method, the combination therapy, or the use, 28 days are counted as one treatment cycle, and the compound of formula (I) or the pharmaceutically acceptable salt thereof and anastrozole are administered daily on days 1-28 in each treatment cycle.

In some specific embodiments of the present disclosure, in the method, the combination therapy, or the use, 28 days are counted as one treatment cycle, and the compound of formula (I) or the pharmaceutically acceptable salt thereof and anastrozole are administered once daily on days 1-28 in each treatment cycle.

In some embodiments of the present disclosure, in the method, the combination therapy, or the use, 28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 3360-5040 mg. In some embodiments, the total dose of the pharmaceutical composition comprising the compound of formula (I) or the pharmaceutically acceptable salt thereof is selected from the group consisting of 3360 mg, 4200 mg, 5040 mg, and a range formed by any two of the aforementioned values. In some embodiments, a total dose of the pharmaceutical composition comprising the compound of formula (I) or the pharmaceutically acceptable salt thereof is preferably 5040 mg.

In some embodiments of the present disclosure, in the method, the combination therapy, or the use, 28 days are counted as one treatment cycle, the administration is performed once daily for 28 consecutive days, and a total dose of the pharmaceutical composition comprising anastrozole administered per treatment cycle is 28-140 mg. In some embodiments of the present disclosure, the total dose of the pharmaceutical composition comprising anastrozole is selected from the group consisting of 28 mg, 56 mg, 84 mg, and a range formed by any two of the aforementioned values. In some embodiments of the present disclosure, the total dose of the pharmaceutical composition comprising anastrozole is preferably 28 mg.

In some embodiments of the present disclosure, in the method, the combination therapy, or the use, 28 days are counted as one treatment cycle, the administration is performed once daily for 28 consecutive days, and a total dose of 5040 mg of the pharmaceutical composition comprising the compound of formula (I) or the pharmaceutically acceptable salt thereof and a total dose of 28 mg of the pharmaceutical composition comprising anastrozole are administered per treatment cycle.

In embodiments of the present disclosure, the treatment cycle described above is repeated as long as the disease is still under control and the administration regimen is clinically tolerable.

In some embodiments of the present disclosure, anastrozole is prepared as a single dose or multiple doses suitable for consecutive daily administration of 1-3 mg or 1-2 mg of anastrozole to a patient; and the pharmaceutical composition comprising the compound of formula (I) or the pharmaceutically acceptable salt thereof is prepared as a single dose or multiple doses suitable for consecutive daily administration of 120 mg, 150 mg, and/or 180 mg based on the compound of formula (I) to a patient.

In some embodiments of the present disclosure, anastrozole is prepared as a single dose suitable for consecutive daily administration of 1 mg of anastrozole to a patient; and the pharmaceutical composition comprising the compound of formula (I) or the pharmaceutically acceptable salt thereof is prepared as multiple doses suitable for consecutive daily administration of 180 mg based on the compound of formula (I) to a patient.

### Combined Pharmaceutical Composition of Compound of Formula (I) or Pharmaceutically Acceptable Salt Thereof and Tamoxifen

In some embodiments of the present disclosure, the present disclosure provides a combined pharmaceutical composition comprising the compound of formula (I) or the pharmaceutically acceptable salt thereof and tamoxifen. In some embodiments of the present disclosure, the present disclosure provides a combined pharmaceutical composition for use in treating or preventing breast cancer, comprising the compound of formula (I) or the pharmaceutically acceptable salt thereof and tamoxifen.

In some embodiments of the present disclosure, the present disclosure provides a combined pharmaceutical composition for use in treating or preventing breast cancer in a patient who has received surgical treatment, comprising the compound of formula (I) or the pharmaceutically acceptable salt thereof and tamoxifen.

In some embodiments of the present disclosure, the combined pharmaceutical composition comprises 10-40 mg or 20-40 mg of tamoxifen or a pharmaceutical composition thereof. Alternatively, the combined pharmaceutical composition is in the form of a single-administration formulation, and the combined pharmaceutical composition comprises 10-40 mg or 20-40 mg of tamoxifen or a pharmaceutical composition thereof. In some embodiments of the present disclosure, the combined pharmaceutical composition comprises 20 mg or 40 mg of tamoxifen or a pharmaceutical composition thereof. In some embodiments of the present disclosure, the combined pharmaceutical composition comprises 20 mg of tamoxifen or a pharmaceutical composition thereof.

In some embodiments of the present disclosure, in the combined pharmaceutical composition, the pharmaceutical composition of tamoxifen is in a single-dose form or a multi-dose form. In some embodiments of the present disclosure, in the combined pharmaceutical composition, the pharmaceutical composition of tamoxifen is in a single-dose form. In some embodiments of the present disclosure, in the combined pharmaceutical composition, the content of tamoxifen is a daily dose or a one-day dose. In some embodiments of the present disclosure, in the combined pharmaceutical composition, the content of tamoxifen is a twice-daily dose or a twice-a-day dose.

In some embodiments of the present disclosure, in the combined pharmaceutical composition, the content of tamoxifen is a twice-daily dose or a twice-a-day dose, with each dose being a single dose or multiple doses, preferably a single dose.

In some embodiments of the present disclosure, the combined pharmaceutical composition comprises a single dose of 10 mg of a pharmaceutical composition of tamoxifen. Alternatively, the combined pharmaceutical composition comprises a single dose of 20 mg of a pharmaceutical composition of tamoxifen.

In some embodiments of the present disclosure, the pharmaceutical composition of tamoxifen comprised in the combined pharmaceutical composition is packaged in a kit, and the kit further comprises an instruction for use of tamoxifen for treating or preventing breast cancer.

In some embodiments of the present disclosure, the pharmaceutical composition of tamoxifen comprised in the combined pharmaceutical composition is packaged in a kit, the kit further comprises an instruction for use of tamoxifen for treating or preventing breast cancer, and the instruction may be the instruction of a commercially available tamoxifen kit.

In some embodiments of the present disclosure, the combined pharmaceutical composition comprises 20-240 mg of the compound of formula (I) or the pharmaceutically acceptable salt thereof based on the compound of formula (I), and 20-40 mg of tamoxifen.

In some embodiments of the present disclosure, the combined pharmaceutical composition comprises 120-180 mg of the compound of formula (I) or the pharmaceutically acceptable salt thereof based on the compound of formula (I), and 20 mg or 40 mg of tamoxifen.

In some embodiments of the present disclosure, the combined pharmaceutical composition comprises a daily dose of 20 mg, 40 mg, 60 mg, 80 mg, 100 mg, 120 mg, 150 mg, 180 mg, or 240 mg of the compound of formula (I) or the pharmaceutically acceptable salt thereof based on the compound of formula (I), and a daily dose of 20 mg of tamoxifen.

In some embodiments of the present disclosure, the combined pharmaceutical composition comprises a daily dose of 120 mg, 150 mg, or 180 mg of the compound of formula (I) or the pharmaceutically acceptable salt thereof based on the compound of formula (I), and a daily dose of 20 mg of tamoxifen.

In some embodiments of the present disclosure, the combined pharmaceutical composition comprises a single dose of 50 mg or 60 mg of a pharmaceutical composition of the compound of formula (I) or the pharmaceutically acceptable salt thereof based on the compound of formula (I), and a single dose of 10 mg of a pharmaceutical composition of tamoxifen.

In some embodiments of the present disclosure, the combined pharmaceutical composition comprises a single dose of 60 mg of a pharmaceutical composition of the compound of formula (I) or the pharmaceutically acceptable salt thereof based on the compound of formula (I), and a single dose of 10 mg of a pharmaceutical composition of tamoxifen.

In some embodiments of the present disclosure, the combined pharmaceutical composition comprises the compound of formula (I) or the pharmaceutically acceptable salt thereof or the pharmaceutical composition thereof and tamoxifen or the pharmaceutical composition thereof in a mass ratio of 1:1 to 200:1, preferably 1:1 to 180:1, 6:1 to 180:1, 6:1 to 150:1, 6:1 to 100:1, 6:1 to 90:1, 6:1 to 10:1, 6:1 to 9:1, or any ratio within the range described above, wherein the amount of the compound of formula (I) or the pharmaceutically acceptable salt thereof or the pharmaceutical composition thereof is based on the mass of the compound of formula (I), and the amount of tamoxifen or the pharmaceutical composition thereof is based on the mass of tamoxifen.

In some embodiments of the present disclosure, the combined pharmaceutical composition is a formulation suitable for administration in a single treatment cycle (e.g., 28 days), and the formulation comprises: a total amount of 1680-5040 mg (e.g., 3360-5040 mg, preferably 3360 mg, 4200 mg, or 5040 mg) of the compound of formula (I) or the pharmaceutically acceptable salt thereof or a pharmaceutical composition thereof; and a total amount of 560-1120 mg (e.g., 560 mg) of tamoxifen or a pharmaceutical composition thereof, wherein the amount of the compound of formula (I) or the pharmaceutically acceptable salt thereof or the pharmaceutical composition thereof is based on the mass of the compound of formula (I), and the amount of tamoxifen or the pharmaceutical composition thereof is based on the mass of tamoxifen. In some preferred embodiments, the compound of formula (I) or the pharmaceutically acceptable salt thereof or the pharmaceutical composition thereof is packaged in a single aliquot or multiple aliquots (e.g., 2, 4, 7, 14, 28 or more aliquots). In some preferred embodiments, tamoxifen or the pharmaceutical composition thereof is packaged in a single aliquot or multiple aliquots (e.g., 2, 4, 7, 14, 28 or more aliquots). In some embodiments, the compound of formula (I) or the pharmaceutically acceptable salt thereof or the pharmaceutical composition thereof and tamoxifen or the pharmaceutical composition thereof are packaged separately or together.

In some embodiments of the present disclosure, the combined pharmaceutical composition is a formulation suitable for administration in a single treatment cycle (e.g., 28 days), and the formulation comprises the compound of formula (I) or the pharmaceutically acceptable salt thereof or a pharmaceutical composition thereof and tamoxifen or a pharmaceutical composition thereof in a mass ratio of 1.5:1 to 180:1, e.g., 6:1 to 180:1, 6:1 to 120:1, 6:1 to 90:1, 6:1 to 60:1, 6:1 to 30:1, 6:1 to 9:1, 7.5:1, or any ratio within the range described above, wherein the amount of the compound of formula (I) or the pharmaceutically acceptable salt thereof or the pharmaceutical composition thereof is based on the mass of the compound of formula (I), and the amount of tamoxifen or the pharmaceutical composition thereof is based on the mass of tamoxifen.

In some embodiments of the present disclosure, in the combined pharmaceutical composition, the compound of formula (I) or the pharmaceutically acceptable salt thereof and tamoxifen may be each in the form of a pharmaceutical composition or be in the form of a pharmaceutical composition together.

In another aspect, the present disclosure further provides a kit comprising: (a) a first pharmaceutical composition comprising the compound of formula (I) or the pharmaceutically acceptable salt thereof described herein; and (b) a second pharmaceutical composition comprising tamoxifen.

In another aspect, the present disclosure further provides a kit comprising a pharmaceutical composition for use in treating or preventing breast cancer, comprising: (a) a first pharmaceutical composition comprising the compound of formula (I) or the pharmaceutically acceptable salt thereof described herein; and (b) a second pharmaceutical composition comprising tamoxifen.

In another aspect, the present disclosure further provides a method for treating or preventing breast cancer, comprising administering to an individual in need thereof a therapeutically effective amount of the compound of formula (I) or the pharmaceutically acceptable salt thereof and tamoxifen, for example, administering to an individual in need thereof a therapeutically effective amount of the combined pharmaceutical composition described above in the present disclosure.

In another aspect, the present disclosure further provides a combination therapy for use in treating an individual with breast cancer, comprising administering to the individual a therapeutically effective amount of the compound of formula (I) or the pharmaceutically acceptable salt thereof alone and a therapeutically effective amount of tamoxifen alone.

In another aspect, the present disclosure further provides use of the compound of formula (I) or the pharmaceutically acceptable salt thereof in combination with tamoxifen for preparing a medicament for treating or preventing breast cancer, for example, use of the combined pharmaceutical composition described above in the present disclosure for preparing a medicament for treating or preventing breast cancer. In some embodiments of the present disclosure, the combined pharmaceutical composition is the combined pharmaceutical composition described herein.

In another aspect, the present disclosure further provides use of the compound of formula (I) or the pharmaceutically acceptable salt thereof in combination with tamoxifen for treating or preventing breast cancer, for example, use of the combined pharmaceutical composition described above in the present disclosure for treating or preventing breast cancer.

In some embodiments of the present disclosure, in the kit, the method, the combination therapy, or the use, the definitions of the compound of formula (I) or the pharmaceutically acceptable salt thereof and tamoxifen are the same as those of the compound of formula (I) or the pharmaceutically acceptable salt thereof and tamoxifen in the combined pharmaceutical composition described herein, for example, in terms of content, dose, form of presence, form of packaging, or the like.

In some embodiments of the present disclosure, in the method, the combination therapy, or the use, the compound of formula (I) or the pharmaceutically acceptable salt thereof and tamoxifen are each in the form of a pharmaceutical composition and can be administered simultaneously, separately, concurrently, sequentially, or at intervals.

In some embodiments of the present disclosure, in the method, the combination therapy, or the use, the compound of formula (I) or the pharmaceutically acceptable salt thereof and tamoxifen each have the same or different treatment cycles. In some specific embodiments of the present disclosure, in the method, the combination therapy, or the use, the compound of formula (I) or the pharmaceutically acceptable salt thereof and tamoxifen have the same treatment cycle, e.g., a 1-week, 2-week, 3-week or 4-week treatment cycle.

In some embodiments of the present disclosure, in the method, the combination therapy, or the use, the content of the compound of formula (I) or the pharmaceutically acceptable salt thereof in the combined pharmaceutical composition is a daily dose, which is administered by administering the compound of formula (I) or the pharmaceutically acceptable salt thereof once daily.

In some embodiments of the present disclosure, in the method, the combination therapy, or the use, the content of the compound of formula (I) or the pharmaceutically acceptable salt thereof in the combined pharmaceutical composition is a daily dose, wherein the compound of formula (I) or the pharmaceutically acceptable salt thereof is administered in a single dose or multiple doses, typically in multiple doses; further, the compound of formula (I) or the pharmaceutically acceptable salt thereof is administered once daily.

In some embodiments of the present disclosure, in the method, the combination therapy, or the use, the compound of formula (I) or the pharmaceutically acceptable salt thereof is administered in a daily dose of 120 mg, in a daily dose of 150 mg, or in a daily dose of 180 mg.

In some embodiments of the present disclosure, in the method, the combination therapy, or the use, the compound of formula (I) or the pharmaceutically acceptable salt thereof in the combined pharmaceutical composition is administered in multiple doses consisting of single doses of 50 mg or 60 mg of the pharmaceutical composition of the compound of formula (I) or the pharmaceutically acceptable salt thereof, preferably in multiple doses consisting of single doses of 60 mg of the pharmaceutical composition of the compound of formula (I) or the pharmaceutically acceptable salt thereof. In some embodiments of the present disclosure, in the method, the combination therapy, or the use, the compound of formula (I) or the pharmaceutically acceptable salt thereof is administered on a regimen of consecutive daily administration.

In some embodiments of the present disclosure, in the method, the combination therapy, or the use, the content of the compound of formula (I) or the pharmaceutically acceptable salt thereof in the combined pharmaceutical composition is a dose per treatment cycle, which is administered by administering the compound of formula (I) or the pharmaceutically acceptable salt thereof daily.

In some embodiments of the present disclosure, in the method, the combination therapy, or the use, the content of tamoxifen in the combined pharmaceutical composition is a daily dose, which is administered by administering tamoxifen twice daily.

In some embodiments of the present disclosure, in the method, the combination therapy, or the use, the content of tamoxifen in the combined pharmaceutical composition is a daily dose, wherein tamoxifen is administered in a single dose or multiple doses, typically in a single dose; further, tamoxifen is administered twice daily.

In some embodiments of the present disclosure, in the method, the combination therapy, or the use, tamoxifen is administered in a daily dose of 20 mg.

In some embodiments of the present disclosure, in the method, the combination therapy, or the use, tamoxifen in the combined pharmaceutical composition is administered in a single dose of 10 mg of the pharmaceutical composition of tamoxifen. In some embodiments of the present disclosure, in the method, the combination therapy, or the use, tamoxifen is administered on a regimen of consecutive daily administration.

In some embodiments of the present disclosure, in the method, the combination therapy, or the use, the content of tamoxifen in the combined pharmaceutical composition is a dose per treatment cycle, which is administered by administering tamoxifen daily.

In some embodiments of the present disclosure, in the method, the combination therapy, or the use, 28 days are counted as one treatment cycle, and the compound of formula (I) or the pharmaceutically acceptable salt thereof and tamoxifen are administered daily on days 1-28 in each treatment cycle.

In some specific embodiments of the present disclosure, in the method, the combination therapy, or the use, 28 days are counted as one treatment cycle, the compound of formula (I) or the pharmaceutically acceptable salt thereof is administered once daily on days 1-28 in each treatment cycle, and tamoxifen is administered twice daily on days 1-28 in each treatment cycle.

In some embodiments of the present disclosure, in the method, the combination therapy, or the use, 28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 3360-5040 mg. In some embodiments, the total dose of the pharmaceutical composition comprising the compound of formula (I) or the pharmaceutically acceptable salt thereof is selected from the group consisting of 3360 mg, 4200 mg, 5040 mg, and a range formed by any two of the aforementioned values. In some embodiments, a total dose of the pharmaceutical composition comprising the compound of formula (I) or the pharmaceutically acceptable salt thereof is preferably 5040 mg.

In some embodiments of the present disclosure, in the method, the combination therapy, or the use, 28 days are counted as one treatment cycle, the administration is performed twice daily for 28 consecutive days, and a total dose of the pharmaceutical composition comprising tamoxifen administered per treatment cycle is 560-1120 mg. In some embodiments of the present disclosure, the total dose of the pharmaceutical composition comprising tamoxifen is selected from the group consisting of 560 mg, 1120 mg, and a range formed by any two of the aforementioned values. In some embodiments of the present disclosure, the total dose of the pharmaceutical composition comprising tamoxifen is preferably 560 mg.

In some embodiments of the present disclosure, in the method, the combination therapy, or the use, 28 days are counted as one treatment cycle, the administration is performed once daily for 28 consecutive days, and a total dose of 5040 mg of the pharmaceutical composition comprising the compound of formula (I) or the pharmaceutically acceptable salt thereof and a total dose of 560 mg of the pharmaceutical composition comprising tamoxifen are administered per treatment cycle.

In embodiments of the present disclosure, the treatment cycle described above is repeated as long as the disease is still under control and the administration regimen is clinically tolerable.

In some embodiments of the present disclosure, tamoxifen is prepared as a single dose or multiple doses suitable for consecutive daily administration of 10-40 mg or 20-40 mg of tamoxifen to a patient; and the pharmaceutical composition comprising the compound of formula (I) or the pharmaceutically acceptable salt thereof is prepared as a single dose or multiple doses suitable for consecutive daily administration of 120 mg, 150 mg, and/or 180 mg based on the compound of formula (I) to a patient.

In some embodiments of the present disclosure, tamoxifen is prepared as a single dose suitable for consecutive daily administration of 10 mg of tamoxifen to a patient; and the pharmaceutical composition comprising the compound of formula (I) or the pharmaceutically acceptable salt thereof is prepared as multiple doses suitable for consecutive daily administration of 180 mg based on the compound of formula (I) to a patient.

In some embodiments of the present disclosure, the method, the combination therapy, or the use is a method, a therapy, or use for adjuvant therapy. In some embodiments of the present disclosure, the method, combination therapy, or use is a method, a therapy, or use for adjuvant therapy of breast cancer. In some embodiments of the present disclosure, the method, the combination therapy, or the use is a method, a therapy, or use for providing adjuvant therapy for a patient with breast cancer.

In some embodiments of the present disclosure, the surgical treatment is selected from the group consisting of radical surgery and breast-conserving surgery. In some embodiments of the present disclosure, the breast-conserving surgery is breast-conserving surgery with no residual invasive carcinoma, ductal carcinoma in situ, or lobular carcinoma in situ at the surgical margin. For example, pathological examination confirms that there is no residual invasive carcinoma, ductal carcinoma in situ, or lobular carcinoma in situ at the surgical margin after breast-conserving surgery.

In some embodiments of the present disclosure, the surgical treatment is selected from the group consisting of radical surgery and breast-conserving surgery with no residual invasive carcinoma, ductal carcinoma in situ, or lobular carcinoma in situ at the surgical margin. In some embodiments of the present disclosure, the radical surgery is selected from the group consisting of modified radical surgery, classical radical surgery, and extended radical surgery. In some embodiments of the present disclosure, the radical surgery for breast cancer consists of two parts: breast management and axillary management. In some embodiments of the present disclosure, there is no recurrence or distant metastasis after the surgical treatment (e.g., there is no evidence of recurrence or distant metastasis after the surgical treatment). In some embodiments of the present disclosure, the surgical treatment is radical resection surgery for breast cancer, or a treatment with no residual invasive carcinoma, ductal carcinoma in situ, or lobular carcinoma in situ at the surgical margin after breast-conserving surgery as confirmed by pathological examination; and there is no evidence of recurrence or distant metastasis after the surgical treatment. In some embodiments of the present disclosure, the surgical treatment is selected from the group consisting of breast-conserving surgery, total mastectomy, and post-mastectomy reconstruction. In some embodiments of the present disclosure, the surgical treatment is a surgical treatment for breast management and/or axillary management.

In some embodiments of the present disclosure, the breast cancer is selected from invasive breast cancer. Preferably, the breast cancer is selected from HR-positive, HER2-negative invasive breast cancer.

In some embodiments of the present disclosure, the breast cancer is selected from early-stage breast cancer.

In some embodiments of the present disclosure, the breast cancer is selected from early-stage invasive breast cancer.

In some embodiments of the present disclosure, the breast cancer is selected from breast cancer with a high risk of recurrence.

In some embodiments of the present disclosure, the breast cancer is selected from hormone receptor (HR)-positive breast cancer.

In some embodiments of the present disclosure, the breast cancer is selected from human epidermal growth factor receptor 2 (HER2)-negative breast cancer.

In some embodiments of the present disclosure, the breast cancer is selected from HR-positive, HER2-negative breast cancer. In some embodiments of the present disclosure, the breast cancer is selected from HR-positive, HER2-negative locally advanced and/or metastatic breast cancer.

In some embodiments of the present disclosure, the breast cancer is selected from breast cancer with a ki-67 index ≥ 20%.

In some embodiments of the present disclosure, the breast cancer is selected from lymph node-positive breast cancer.

In some embodiments of the present disclosure, the breast cancer is selected from breast cancer with a high risk of recurrence in a patient who has received surgical treatment.

In some embodiments of the present disclosure, the breast cancer is selected from early-stage breast cancer in a patient who has received surgical treatment.

In some embodiments of the present disclosure, the breast cancer is selected from HR-positive, HER2-negative breast cancer in a patient who has received surgical treatment.

In some embodiments of the present disclosure, the breast cancer is selected from breast cancer with a ki-67 index ≥ 20% in a patient who has received surgical treatment.

In some embodiments of the present disclosure, the breast cancer is selected from lymph node-positive breast cancer in a patient who has received surgical treatment.

In some embodiments of the present disclosure, the breast cancer is selected from HR-positive, HER2-negative breast cancer with a ki-67 index ≥ 20%.

In some embodiments of the present disclosure, the breast cancer is selected from lymph node-positive, HR-positive, HER2-negative breast cancer.

In some embodiments of the present disclosure, the breast cancer is selected from HR-positive, HER2-negative breast cancer with a high risk of recurrence.

In some embodiments of the present disclosure, the breast cancer is selected from HR-positive, HER2-negative early-stage breast cancer.

In some embodiments of the present disclosure, the breast cancer is selected from lymph node-positive breast cancer with a ki-67 index ≥ 20%.

In some embodiments of the present disclosure, the breast cancer is selected from breast cancer with a ki-67 index ≥ 20% and a high risk of recurrence.

In some embodiments of the present disclosure, the breast cancer is selected from early-stage breast cancer with a ki-67 index ≥ 20%.

In some embodiments of the present disclosure, the breast cancer is selected from lymph node-positive breast cancer with a high risk of recurrence.

In some embodiments of the present disclosure, the breast cancer is selected from lymph node-positive early-stage breast cancer.

In some embodiments of the present disclosure, the breast cancer is selected from early-stage breast cancer with a high risk of recurrence.

In some embodiments of the present disclosure, the breast cancer is selected from HR-positive, HER2-negative early-stage breast cancer in a patient who has received surgical treatment.

In some embodiments of the present disclosure, the breast cancer is selected from HR-positive, HER2-negative breast cancer with a ki-67 index ≥ 20% in a patient who has received surgical treatment.

In some embodiments of the present disclosure, the breast cancer is selected from lymph node-positive, HR-positive, HER2-negative breast cancer in a patient who has received surgical treatment.

In some embodiments of the present disclosure, the breast cancer is selected from HR-positive, HER2-negative breast cancer with a high risk of recurrence in a patient who has received surgical treatment.

In some embodiments of the present disclosure, the breast cancer is selected from lymph node-positive breast cancer with a ki-67 index ≥ 20% in a patient who has received surgical treatment.

In some embodiments of the present disclosure, the breast cancer is selected from lymph node-positive breast cancer with a high risk of recurrence in a patient who has received surgical treatment.

In some embodiments of the present disclosure, the breast cancer is selected from lymph node-positive early-stage breast cancer in a patient who has received surgical treatment.

In some embodiments of the present disclosure, the breast cancer is selected from breast cancer with a ki-67 index ≥ 20% and a high risk of recurrence in a patient who has received surgical treatment.

In some embodiments of the present disclosure, the breast cancer is selected from early-stage breast cancer with a ki-67 index ≥ 20% in a patient who has received surgical treatment.

In some embodiments of the present disclosure, the breast cancer is selected from early-stage breast cancer with a high risk of recurrence in a patient who has received surgical treatment.

In some embodiments of the present disclosure, the breast cancer is selected from HR-positive, HER2-negative breast cancer with a ki-67 index ≥ 20% and a high risk of recurrence.

In some embodiments of the present disclosure, the breast cancer is selected from HR-positive, HER2-negative early-stage breast cancer with a ki-67 index ≥ 20%.

In some embodiments of the present disclosure, the breast cancer is selected from HR-positive, HER2-negative early-stage breast cancer with a high risk of recurrence.

In some embodiments of the present disclosure, the breast cancer is selected from lymph node-positive, HR-positive, HER2-negative breast cancer with a ki-67 index ≥ 20%.

In some embodiments of the present disclosure, the breast cancer is selected from lymph node-positive, HR-positive, HER2-negative breast cancer with a high risk of recurrence.

In some embodiments of the present disclosure, the breast cancer is selected from lymph node-positive, HR-positive, HER2-negative early-stage breast cancer.

In some embodiments of the present disclosure, the breast cancer is selected from lymph node-positive early-stage breast cancer with a ki-67 index ≥ 20%.

In some embodiments of the present disclosure, the breast cancer is selected from lymph node-positive breast cancer with a ki-67 index ≥ 20% and a high risk of recurrence.

In some embodiments of the present disclosure, the breast cancer is selected from early-stage breast cancer with a ki-67 index ≥ 20% and a high risk of recurrence.

In some embodiments of the present disclosure, the breast cancer is selected from lymph node-positive early-stage breast cancer with a high risk of recurrence.

In some embodiments of the present disclosure, the breast cancer is selected from HR-positive, HER2-negative breast cancer with a ki-67 index ≥ 20% and a high risk of recurrence in a patient who has received surgical treatment.

In some embodiments of the present disclosure, the breast cancer is selected from HR-positive, HER2-negative early-stage breast cancer with a ki-67 index ≥ 20% in a patient who has received surgical treatment.

In some embodiments of the present disclosure, the breast cancer is selected from lymph node-positive, HR-positive, HER2-negative breast cancer with a ki-67 index ≥ 20% in a patient who has received surgical treatment.

In some embodiments of the present disclosure, the breast cancer is selected from lymph node-positive early-stage breast cancer with a ki-67 index ≥ 20% in a patient who has received surgical treatment.

In some embodiments of the present disclosure, the breast cancer is selected from lymph node-positive breast cancer with a ki-67 index ≥ 20% and a high risk of recurrence in a patient who has received surgical treatment.

In some embodiments of the present disclosure, the breast cancer is selected from lymph node-positive early-stage breast cancer with a high risk of recurrence in a patient who has received surgical treatment.

In some embodiments of the present disclosure, the breast cancer is selected from early-stage breast cancer with a ki-67 index ≥ 20% and a high risk of recurrence in a patient who has received surgical treatment.

In some embodiments of the present disclosure, the breast cancer is selected from HR-positive, HER2-negative early-stage breast cancer with a high risk of recurrence in a patient who has received surgical treatment.

In some embodiments of the present disclosure, the breast cancer is selected from lymph node-positive, HR-positive, HER2-negative early-stage breast cancer in a patient who has received surgical treatment.

In some embodiments of the present disclosure, the breast cancer is selected from lymph node-positive, HR-positive, HER2-negative breast cancer with a high risk of recurrence in a patient who has received surgical treatment.

In some embodiments of the present disclosure, the breast cancer is selected from lymph node-positive, HR-positive, HER2-negative breast cancer with a ki-67 index ≥ 20% and a high risk of recurrence.

In some embodiments of the present disclosure, the breast cancer is selected from lymph node-positive, HR-positive, HER2-negative early-stage breast cancer with a ki-67 index ≥ 20%.

In some embodiments of the present disclosure, the breast cancer is selected from lymph node-positive, HR-positive, HER2-negative early-stage breast cancer with a high risk of recurrence.

In some embodiments of the present disclosure, the breast cancer is selected from lymph node-positive early-stage breast cancer with a ki-67 index ≥ 20% and a high risk of recurrence.

In some embodiments of the present disclosure, the breast cancer is selected from HR-positive, HER2-negative early-stage breast cancer with a ki-67 index ≥ 20% and a high risk of recurrence.

In some embodiments of the present disclosure, the breast cancer is selected from lymph node-positive, HR-positive, HER2-negative breast cancer with a ki-67 index ≥ 20% and a high risk of recurrence in a patient who has received surgical treatment.

In some embodiments of the present disclosure, the breast cancer is selected from lymph node-positive, HR-positive, HER2-negative early-stage breast cancer with a ki-67 index ≥ 20% in a patient who has received surgical treatment.

In some embodiments of the present disclosure, the breast cancer is selected from lymph node-positive early-stage breast cancer with a ki-67 index ≥ 20% and a high risk of recurrence in a patient who has received surgical treatment.

In some embodiments of the present disclosure, the breast cancer is selected from lymph node-positive, HR-positive, HER2-negative early-stage breast cancer with a high risk of recurrence in a patient who has received surgical treatment.

In some embodiments of the present disclosure, the breast cancer is selected from lymph node-positive, HR-positive, HER2-negative early-stage breast cancer with a ki-67 index ≥ 20% and a high risk of recurrence.

In some embodiments of the present disclosure, the breast cancer is selected from HR-positive, HER2-negative early-stage breast cancer with a ki-67 index ≥ 20% and a high risk of recurrence in a patient who has received surgical treatment.

In some embodiments of the present disclosure, the breast cancer is selected from lymph node-positive, HR-positive, HER2-negative early-stage breast cancer with a ki-67 index ≥ 20% and a high risk of recurrence in a patient who has received surgical treatment.

In some embodiments of the present disclosure, the breast cancer is selected from the group consisting of postmenopausal and premenopausal/perimenopausal breast cancer. In some embodiments of the present disclosure, the breast cancer is breast cancer in a patient who has received radical resection surgery for breast cancer or has no residual invasive carcinoma, ductal carcinoma in situ, or lobular carcinoma in situ at the surgical margin after breast-conserving surgery as confirmed by pathological examination, and who has no evidence of recurrence or distant metastasis after surgery.

In some embodiments of the present disclosure, the breast cancer is selected from the group consisting of postmenopausal and premenopausal/perimenopausal breast cancer in a patient who has previously received bilateral ovariectomy.

In some embodiments of the present disclosure, the breast cancer is selected from postmenopausal breast cancer in a patient aged ≥ 60 years.

In some embodiments of the present disclosure, the breast cancer is selected from postmenopausal breast cancer in a patient aged < 60 years with natural menopause ≥ 12 months.

In some embodiments of the present disclosure, the breast cancer is selected from premenopausal/perimenopausal breast cancer in a patient receiving LHRH agonist treatment.

In some embodiments of the present disclosure, being lymph node-positive refers to the presence of axillary lymph node metastasis.

In some embodiments of the present disclosure, being lymph node-positive refers to the presence of lymph node metastasis in the ipsilateral side of the breast lesion.

In some embodiments of the present disclosure, being lymph node-positive refers to the presence of ≥ 4 axillary lymph node metastases.

In some embodiments of the present disclosure, being lymph node-positive refers to the presence of 1-3 axillary lymph node metastases and a primary lesion with a diameter of ≥ 5 cm as determined by pathological examination; or the presence of 1-3 axillary lymph node metastases and a primary lesion with histological grade III; or the presence of 1-3 axillary lymph node metastases and residual invasive carcinoma in the primary breast lesion or the ipsilateral axillary lymph node after neoadjuvant therapy.

In some embodiments of the present disclosure, the patient with breast cancer has received neoadjuvant therapy before surgical treatment. In some embodiments of the present disclosure, a regimen for the neoadjuvant therapy includes an EC-T regimen (epirubicin plus cyclophosphamide, followed by docetaxel), a TEC regimen (docetaxel, epirubicin, and cyclophosphamide), a TC regimen (docetaxel plus cyclophosphamide), and/or a TCb regimen (docetaxel plus carboplatin, combined with trastuzumab and pertuzumab).

In some embodiments of the present disclosure, the patient with breast cancer has received adjuvant therapy after surgical treatment and before the implementation of the method, the combination therapy, or the use of the present disclosure, and shows no recurrence.

In some embodiments of the present disclosure, the patient with breast cancer has received adjuvant therapy with an endocrine drug after surgical treatment and before the implementation of the method, the combination therapy, or the use of the present disclosure, and shows no recurrence.

In some embodiments of the present disclosure, the patient with breast cancer has received neoadjuvant therapy after surgical treatment and before the implementation of the method, the combination therapy, or the use of the present disclosure, and shows no recurrence.

In some embodiments of the present disclosure, the patient with breast cancer has received neoadjuvant therapy after a surgical treatment and before the implementation of the method, the combination therapy, or the use of the present disclosure, and shows no recurrence, and a regimen for the neoadjuvant therapy includes an EC-T regimen (epirubicin plus cyclophosphamide, followed by docetaxel), a TEC regimen (docetaxel, epirubicin, and cyclophosphamide), a TC regimen (docetaxel plus cyclophosphamide), and/or a TCb regimen (docetaxel plus carboplatin, combined with trastuzumab and pertuzumab).

In some embodiments of the present disclosure, the breast cancer patient has completed adjuvant therapy and/or neoadjuvant therapy in accordance with guidelines for diagnosis and treatment after surgical treatment and before the implementation of the method, the combination therapy, or the use of the present disclosure, and the previous treatment for breast cancer needs to meet the following conditions: (1) the subject who received neoadjuvant therapy must have received at least 4 cycles of treatment, and the chemotherapy regimen must include taxanes; (2) the interval from radical breast surgery or breast-conserving surgery to the date of randomization shall not exceed 14 months; (3) the interval from the date of the last administration of adjuvant therapy to the date of randomization shall be not less than 21 days; (4) the interval from the date of the last radiotherapy of adjuvant radiotherapy to the date of randomization shall be not less than 14 days; (5) cumulative endocrine therapy of no more than 12 weeks is permitted from the last non-endocrine therapy (including surgery, radiotherapy, and chemotherapy) to randomization (the duration of LHRH agonist therapy is not limited).

In some embodiments of the present disclosure, being HR-positive includes being estrogen receptor (ER)-positive and/or progesterone receptor (PR)-positive, and is defined as follows: the proportion of tumor cells with positive staining in all tumor cells is ≥ 10%.

In some embodiments of the present disclosure, being HER2-negative is defined as follows: HER2 is 0/1+ as detected by immunohistochemical staining (IHC); if HER2 is 2+ as detected, fluorescence in situ hybridization (FISH) should be performed to confirm that the result is negative, or FISH assay is performed alone and the result is negative.

The "date of randomization" described in the present disclosure refers to the time at which a subject is randomly allocated to receive the drug.

As used herein, "adjuvant therapy" refers to the administration of one or more drugs to a patient after surgical resection of one or more cancerous tumors (where all detectable and resectable diseases (e.g., cancer) have been removed from the patient, but there is still a statistical risk of recurrence due to the occult disease) with the purpose of reducing the likelihood or severity of disease recurrence or delaying the onset of the biological manifestations of disease recurrence.

The "Ki67 antigen", or abbreviated as "Ki67" (also referred to as an antigen recognized by the monoclonal antibody Ki-67), described herein refers to a nucleoprotein encoded by the MKI67 gene that is expressed in all stages of the cell cycle other than G0 phase and has been reported as an independent prognostic factor in early-stage breast cancer (Dowsett et al., 2011). In HR+ breast cancer, patients with high Ki67 levels have been shown to have higher rates of disease recurrence while receiving adjuvant endocrine therapy after surgery. In the BIG 1-98 study (Viale et al., 2008), patients with HR+ early-stage breast cancer involving their axillary lymph nodes and low (≤ 11%) baseline levels of Ki67 who received letrozole had a 4-year disease-free survival rate of 93%. In contrast, patients with higher Ki67 values (> 11%) had a 4-year disease-free survival rate of 85%. Currently, there is no consensus regarding the precise baseline Ki67 level to discriminate between patients with a higher or lower risk of disease recurrence while receiving adjuvant endocrine therapy. However, most of the panel of experts of the St Gallen International Expert Consensus on the Primary Therapy of Early Breast Cancer 2015 was prepared to accept a Ki67 threshold in the range of 20% to 29% (Vasconcelos et al., 2016) as an indicator for the high-risk group suitable for adjuvant chemotherapy.

The active components in the pharmaceutical combination of the present disclosure can each be formulated independently, or some or all of the active components are co-formulated with a pharmaceutically acceptable carrier and/or excipient. The pharmaceutical combination of the present disclosure may further comprise an additional therapeutic agent. In some embodiments of the present disclosure, the additional therapeutic agent may be a therapeutic agent known in the art for cancer, preferably a therapeutic agent for breast cancer.

In some embodiments of the present disclosure, the treatment cycle is 28 days. The amount of the compound of formula (I) or the pharmaceutically acceptable salt thereof administered can be determined according to the severity of the disease, the response of the disease, any treatment-related toxicity, and the age and health of a patient.

The compound of formula (I) or the pharmaceutically acceptable salt thereof can be administered via multiple routes of administration including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, intraperitoneal, and intrathecal administrations. In a specific embodiment, the compound of formula (I) or the pharmaceutically acceptable salt thereof is administered orally.

In some embodiments of the present disclosure, the compound of formula (I) or the pharmaceutically acceptable salt thereof is administered based on a regimen of consecutive daily oral administration. The compound of formula (I) or the pharmaceutically acceptable salt thereof can be administered once or multiple times daily. In some embodiments of the present disclosure, the compound of formula (I) or the pharmaceutically acceptable salt thereof is administered once daily. The compound of formula (I) or the pharmaceutically acceptable salt thereof may also be administered in a single-dose form or a multi-dose form. In one embodiment, the compound of formula (I) or the pharmaceutically acceptable salt thereof is administered in multiple doses once daily.

In some embodiments of the present disclosure, the compound of formula (I) or the pharmaceutically acceptable salt thereof is administered in the form of a multi-dose oral solid formulation once daily. In one embodiment, the compound of formula (I) or the pharmaceutically acceptable salt thereof is administered in multiple doses once daily.

The mode of administration can be determined comprehensively according to the activity and toxicity of the medicament, the tolerance of a patient, and the like.

### Compound of Formula (I) or Pharmaceutically Acceptable Salt Thereof

As used herein, the pharmaceutically acceptable salt of the compound of formula (I) may be a maleate of the compound of formula (I) (e.g., a monomaleate of the compound of formula (I)).

The dosage of the compound of formula (I) or the pharmaceutically acceptable salt thereof referred to herein is based on the mass of a molecule of the compound of formula (I), unless otherwise stated. For example, a dose of 50 mg or 60 mg refers to a molecule of the compound of formula (I) having a mass of 50 mg or 60 mg.

The compound of formula (I) or the pharmaceutically acceptable salt thereof used herein may be prepared by methods known in the art, for example, by referring to the method described in WO2016141881.

### Pharmaceutical Composition of Compound of Formula (I) or Pharmaceutically Acceptable Salt Thereof

In some embodiments of the present disclosure, a single dose of the pharmaceutical composition of the compound of formula (I) or the pharmaceutically acceptable salt thereof may be 50 mg or 60 mg based on the compound of formula (I).

In some embodiments of the present disclosure, a single dose of the pharmaceutical composition of the compound of formula (I) or the pharmaceutically acceptable salt thereof is 60 mg.

The mode of administration can be determined comprehensively according to the activity and toxicity of the medicament, the tolerance of a patient, and the like.

In some embodiments of the present disclosure, the pharmaceutical composition comprising the compound of formula (I) or the pharmaceutically acceptable salt thereof further comprises a pharmaceutically acceptable excipient. The pharmaceutically acceptable excipient includes a filler, an absorbent, a wetting agent, a binder, a disintegrant, a lubricant, and the like. In some embodiments of the present disclosure, the pharmaceutical composition includes, but is not limited to formulations suitable for oral, parenteral, and local administrations; in some embodiments, the pharmaceutical composition is a formulation suitable for oral administration; in some embodiments, the pharmaceutical composition is a solid formulation suitable for oral administration; in some embodiments, the pharmaceutical composition includes, but is not limited to a tablet and a capsule.

In some embodiments of the present disclosure, the pharmaceutical composition is a solid pharmaceutical combination. In some embodiments of the present disclosure, the pharmaceutical composition is a capsule. In some embodiments of the present disclosure, the pharmaceutical composition of the compound of formula (I) or the pharmaceutically acceptable salt thereof is a solid pharmaceutical composition of the compound of formula (I). In some embodiments of the present disclosure, the pharmaceutical composition of the compound of formula (I) or the pharmaceutically acceptable salt thereof is a capsule of the compound of formula (I).

The pharmaceutical composition of the compound of formula (I) or the pharmaceutically acceptable salt thereof of the present disclosure can be manufactured using methods well known in the art, such as conventional mixing, dissolving, granulating, dragee-making, levigating, emulsifying, and lyophilizing.

A solid oral composition can be prepared by conventional mixing, filling, or tableting. For example, the solid oral composition can be obtained by the following method: mixing the active compounds with solid excipients, optionally grinding the resulting mixture, adding additional suitable excipients if desired, and processing the mixture into granules to obtain the core parts of tablets or dragees. Suitable excipients include, but are not limited to: binders, diluents, disintegrants, lubricants, glidants, sweeteners, flavoring agents, and the like.

### Letrozole

As used herein, letrozole is known by the chemical name of 1-[bis(4-cyanophenyl)methyl]-1,2,4-triazole, which has the following structural formula:

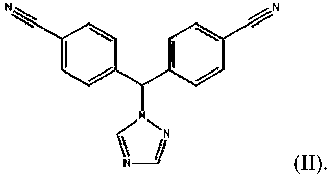

### Pharmaceutical Composition of Letrozole

In some embodiments of the present disclosure, the pharmaceutical composition of letrozole further comprises a pharmaceutically acceptable excipient. In some embodiments, the pharmaceutically acceptable excipient includes a filler, an absorbent, a wetting agent, a binder, a diluent, a disintegrant, a lubricant, a glidant, a sweetener, a flavoring agent, or the like.

In some embodiments of the present disclosure, the pharmaceutical composition is a solid pharmaceutical composition. In some embodiments of the present disclosure, the pharmaceutical composition is a tablet. In some embodiments of the present disclosure, the pharmaceutical composition is an oral tablet.

In some embodiments of the present disclosure, a single dose of the pharmaceutical composition of letrozole is 2.5 mg.

### Anastrozole

As used herein, anastrozole is known by the chemical name of a,a,a',a'-tetramethyl-5-(1*H*-1,2,4-triazol-1-methyl)-1,3-benzenediacetonitrile, which has the following structural formula:

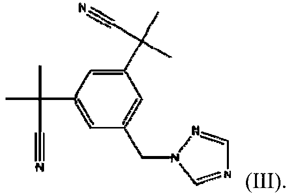

### Pharmaceutical Composition of Anastrozole

In some embodiments of the present disclosure, the pharmaceutical composition of anastrozole further comprises a pharmaceutically acceptable excipient. In some embodiments, the pharmaceutically acceptable excipient includes a filler, an absorbent, a wetting agent, a binder, a diluent, a disintegrant, a lubricant, a glidant, a sweetener, a flavoring agent, or the like.

In some embodiments of the present disclosure, the pharmaceutical composition is a solid pharmaceutical composition. In some embodiments of the present disclosure, the pharmaceutical composition is a tablet. In some embodiments of the present disclosure, the pharmaceutical composition is an oral tablet.

In some embodiments of the present disclosure, a single dose of the pharmaceutical composition of anastrozole is 1 mg.

### Tamoxifen

As used herein, tamoxifen is known by the chemical name (*Z*)-2-[4-(1,2-diphenyl-1-butene)phenoxy]-*N,N-*dimethylethanamine, which has the following structural formula:

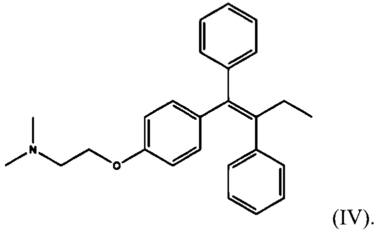

### Pharmaceutical Composition of Tamoxifen

In some embodiments of the present disclosure, the pharmaceutical composition of tamoxifen further comprises a pharmaceutically acceptable excipient. In some embodiments, the pharmaceutically acceptable excipient includes a filler, an absorbent, a wetting agent, a binder, a diluent, a disintegrant, a lubricant, a glidant, a sweetener, a flavoring agent, or the like.

In some embodiments of the present disclosure, the pharmaceutical composition is a solid pharmaceutical composition. In some embodiments of the present disclosure, the pharmaceutical composition is a tablet. In some embodiments of the present disclosure, the pharmaceutical composition is an oral tablet.

In some embodiments of the present disclosure, a single dose of the pharmaceutical composition of tamoxifen is 10 mg.

### Mode of Administration

The content below is not intended to limit the mode of administration of the combined pharmaceutical composition of the present disclosure.

The active ingredients in the combined pharmaceutical composition of the present disclosure can each be administered independently, or some or all of the active ingredients are co-administered in various proper routes, including, but not limited to, oral administration or parenteral administration (via intravenous, intramuscular, topical, or subcutaneous routes). In some embodiments of the present disclosure, the active ingredients in the combined pharmaceutical composition of the present disclosure can each be administered independently, or some or all of the active ingredients are co-administered orally.

The active ingredients in the combined pharmaceutical composition disclosed herein can each be formulated independently in suitable dosage forms, or some or all of the components are co-formulated in a suitable dosage form including, but not limited to, tablet, lozenge, pill, capsule (e.g., hard capsule, soft capsule, enteric capsule, and microcapsule), elixir, granule, syrup, injection (intramuscular injection, intravenous injection, and intraperitoneal injection), granule, emulsion, suspension, solution, dispersant, and dosage forms of sustained-released preparations for oral or non-oral administration.

### Technical Effects

Generally, use of the combined pharmaceutical composition described above of the present disclosure will provide:
(1) better efficacy in reducing the growth of tumors or even eliminating the tumors as compared with administering any drug of the combination alone;
(2) reduced dosage as compared with administering any drug of the combination alone;
(3) treatment that is well tolerated in patients with fewer adverse reactions and/or complications as compared with administering any drug alone;
(4) a better disease control rate in patients treated;
(5) a longer survival (e.g., median survival time, progression-free survival, or overall survival) in patients treated;
(6) a longer survival (e.g., median survival time, progression-free survival, or overall survival) in patients treated as compared with standard chemotherapy;
(7) a longer duration of response (DOR); and/or
(8) better activity in treating tumors or proliferative diseases as compared with administering any drug of the combination alone, demonstrating a better anti-tumor synergistic effect.

The "clinical benefits" of the combined pharmaceutical composition of the present disclosure include, but are not limited to: prolonged progression-free survival (PFS), prolonged overall survival (OS), improved objective response rate (ORR), improved disease control rate (DCR), reduced number and/or degree of adverse reactions, decreased distant metastasis rate, decreased local control rate, and the like for clinical patients. For example, the combined pharmaceutical composition of the present disclosure can significantly prolong the distant relapse free survival, prolong the survival of patients, and particularly prolong the survival of patients without invasive cancer.

### Definitions and Description

As used herein, the term "combined pharmaceutical composition" refers to a combination of two or more active ingredients (administered as the respective active ingredients themselves, or as their respective derivatives such as pharmaceutically acceptable salts or esters, prodrugs, or compositions) that are administered simultaneously or sequentially, and does not mean that the two or more active ingredients must be contained in the same medicament or formulation. As used herein, the terms "combined pharmaceutical composition" and "pharmaceutical combination" are used interchangeably.

The word "comprise", and variants thereof such as "comprises" or "comprising", and equivalents thereof shall be interpreted in an open, non-exclusive sense, i.e., "includes, but is not limited to", indicating that in addition to the listed elements, components, and procedures, other unspecified elements, components, and procedures may also be encompassed.

The term "patient" or "individual/subject" refers to a mammal, e.g., a primate (human, macaque, chimpanzee, etc.), rodent (mouse, rat, rabbit, etc.), feline, canine, etc., preferably a human. In some embodiments of the present disclosure, the patient and the individual are patients who have failed or lack a standard treatment.

The term "pharmaceutically acceptable" refers to a carrier, an excipient, or an auxiliary material that is used in the preparation of a pharmaceutical composition. The carrier, the excipient, or the auxiliary material is generally safe, non-toxic, and not biologically and otherwise undesirable, and inclusion of the substance is acceptable for pharmaceutical use in humans.

The term "therapeutically effective amount" means an amount of a compound that, when administered to a human for treating a disease, is sufficient to treat the disease.

The term "treat", "treating", or "treatment" refers to administering the compound or the formulation described herein to ameliorate, alleviate, or eliminate a disease or one or more symptoms related to the disease, and includes: (i) inhibiting the disease or disease state, i.e., arresting or delaying its progression; and (ii) alleviating the disease or disease state, i.e., causing regression of the disease or disease state.

The term "prevent", "preventing", or "prevention" means administering the compound or formulation described herein to prevent a disease or one or more symptoms related to the disease, and includes preventing the occurrence of the disease or disease state in a mammal, particularly when such a mammal is predisposed to the disease state but has not yet been diagnosed with it.

The use of alternatives (e.g., "or") shall be understood to refer to any one, two, or any combination of the alternatives. As used herein, the indefinite article "a" or "an" shall be interpreted as "one or more" of any listed or enumerated components.

The term "pharmaceutically acceptable excipient" refers to those excipients that do not have a significant irritating effect on an organic entity and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The terms "administer", "administration", and "administering" refer to physically introducing the composition comprising a therapeutic agent to an individual using any one of a variety of methods and delivery systems known to those skilled in the art. In certain embodiments, the administration is oral administration.

The term "daily dose" refers to a dose administered to a patient per day. The term "one-day dose" refers to a dose administered to a patient on one day.

The term "single dose" or "unit formulation" refers to the smallest unit of packaging of a pharmaceutical product comprising a certain amount of active ingredients; for example, in a box of seven capsules, each capsule is a single dose or a unit formulation; in a box of seven tablets, each tablet is a single dose or a unit formulation.

The term "multiple dose" consists of multiple single doses. As used herein, "combined use" or "use in combination" means that two or more active substances may be administered to a subject simultaneously, concurrently, or sequentially in any order as a single formulation.

The term "pharmaceutical composition" refers to a mixture consisting of one or more active ingredients or the pharmaceutical combination thereof of the present disclosure and a pharmaceutically acceptable excipient. The pharmaceutical composition is intended to facilitate the administration of the compound or the pharmaceutical combination thereof of the present disclosure to an individual.

The terms "day", "daily", and the like, when referred to in an administration regimen, refer to the time within a calendar day that starts at midnight and ends at the next midnight.

The term "resection" refers to surgical removal of malignant tissues specific to breast cancer from a patient. According to one embodiment, the resection refers to the removal of malignant tissue such that the presence of residue malignant tissue is undetectable in the patient using available methods. According to another embodiment of the present invention, resection refers to the removal of breast cancer such that the presence of residual breast cancer is undetectable in the patient.

The term "radical surgery" refers to radical resection surgery for breast cancer, including modified radical surgery, classical radical surgery, or extended radical surgery. The term "modified radical surgery" refers to total mastectomy (with preservation of the pectoralis major and minor muscles) + axillary lymph node dissection. The term "classical radical surgery" refers to total mastectomy (with resection of the pectoralis major and minor muscles) + axillary lymph node dissection. The term "extended radical surgery" refers to total mastectomy (with resection of the pectoralis major and minor muscles) + combined axillary and internal mammary lymph node dissection. The term "early-stage" refers to cancer that may have spread to nearby lymph nodes but not to distal parts of the body.

The term "distant relapse free survival" refers to the time from the date of randomization to the first occurrence of distant recurrence or death from any cause.

The term "invasive disease-free survival" refers to the time from the date of randomization to the first occurrence of the following events, including ipsilateral or contralateral recurrent invasive breast cancer, regional or distant recurrent invasive breast cancer, second primary malignancy at a non-breast site, or death from any cause.

The term "overall survival" refers to the time from the date of randomization to the date of death from any cause.

The term "date of randomization" refers to the time at which a subject is randomly allocated to receive the drug.

The term "high risk of recurrence" refers to the presence of lymph node metastasis in the ipsilateral axilla as indicated by postoperative pathological examination of the breast lesion. The "high risk of recurrence" and "high risk" described herein are considered equivalent and are used interchangeably.

As used herein, the terms "endocrine drug" and "endocrine therapeutic agent" are used interchangeably and refer to drugs used for the treatment of endocrine diseases, including, but not limited to aromatase inhibitors, estrogen receptor antagonists, pituitary gonadotropin-releasing hormone analogues, or the like.

As used herein, unless otherwise specified clearly in the context, singular terms encompass plural referents, and vice versa. Similarly, unless the context specifies clearly otherwise, the word "or" is intended to include "and", and vice versa.

Unless otherwise stated herein, parameter values representing amounts of ingredients or physicochemical properties or reaction conditions and the like are to be understood as being modified in all cases by the term "about". When the term "about" is used to describe the present application, the term "about" indicates that there is an error value; for example, it means varying within a range of ± 5%, e.g., ± 1% or + 0.1%, of a particular value.

All patents, patent applications, and other identified publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### Examples

The following specific examples are intended to allow those skilled in the art to clearly understand and implement the present disclosure. These specific examples should not be considered as limitations on the scope of the present disclosure, but merely as exemplary descriptions and representatives of the present disclosure.

### Experimental Example 1

In this study, a randomized, double-blind, parallel-controlled, and multicenter trial design was adopted. Subjects meeting the criteria were grouped according to a random ratio of 2:1 to receive treatment with the capsule of the compound of formula (I) in combination with the endocrine therapy (test group) and the placebo capsule in combination with the endocrine therapy (control group), respectively.

### 1.1. Inclusion criteria:

Subjects who met all of the following inclusion criteria could be enrolled in this trial:
1) Subjects with voluntary participation, signed informed consent, and good compliance;
2) Aged 18-75 years (when signing the informed consent); ECOG PS score: 0-1;
3) Postmenopausal or premenopausal/perimenopausal female patients;
4) Having received radical resection surgery for breast cancer; or has no residual invasive carcinoma, ductal carcinoma in situ, or lobular carcinoma in situ at the surgical margin after breast-conserving surgery as confirmed by pathological examination; and has no evidence of recurrence or distant metastasis after the surgery;
5) Being diagnosed as HR-positive, HER2-negative invasive breast cancer by pathological examination;
6) The presence of lymph node metastasis in the ipsilateral axilla as indicated by postoperative pathological examination of the breast lesion;
7) The subject has completed adjuvant and/or neoadjuvant chemotherapy in accordance with guidelines for diagnosis and treatment before participating in the study;
8) With good main organ functions meeting the following criteria:

Blood routine examination should meet the following criteria (no blood transfusion and no correction using hematopoietic stimulating drugs within 7 days before screening):
a) Hemoglobin (HB) ≥ 90 g/L;
b) Absolute value of neutrophil count (NEUT) ≥ 1.5 × 10⁹/L;
c) Platelet count (PLT) ≥ 100 × 10⁹/L.

Blood biochemical examination should meet the following criteria (no corrective treatment used within 7 days before screening):
a) Total bilirubin (TBIL) ≤ 1.5 times of upper limit of normal (ULN);
b) Alanine aminotransferase (ALT) and aspartate aminotransferase (AST) ≤ 1.5 × ULN;
c) Serum creatinine (Cr) ≤ 1.5 × ULN or creatinine clearance rate (Ccr) ≥ 60 mL/min.

Blood coagulation function examination should meet the following criteria (not having received anticoagulant therapy):
a) Prothrombin time (PT) ≤ 1.5 × ULN;
b) Activated partial thromboplastin time (APTT) ≤ 1.5 × ULN;
c) International normalized ratio (INR) ≤ 1.5 × ULN.

Heart color ultrasound evaluation: left ventricular ejection fraction (LVEF) ≥ 50%.

9) Female subjects of child-bearing age should agree to take contraceptive measures (such as intrauterine devices, contraceptives, or condoms) during the study and within 6 months after the study; serum pregnancy test results should be negative within 7 days before the study enrollment, and the subjects must not be breastfeeding.

### 1.2 Study drugs

Capsules of the compound of formula (I): strength: 60 mg.
Letrozole tablets: strength: 2.5 mg.
Anastrozole tablets: strength: 1 mg.
Tamoxifen tablets: strength: 10 mg.

### 1.3 Administration regimen

### (1) Compound of formula (I):

Capsules of the compound of formula (I): 120 mg, 150 mg, or 180 mg (based on the mass of compound of formula (I)), oral administration at fasting, once daily, with 28 consecutive days of administration counted as one treatment cycle.

(2) Endocrine drugs: one of letrozole, anastrozole and tamoxifen may be selected, and the specific administration regimen is as follows:
Letrozole tablets: 2.5 mg, oral administration, once daily, with 28 consecutive days of administration counted as one treatment cycle.
Anastrozole tablets: 1 mg, oral administration, once daily, with 28 consecutive days of administration counted as one treatment cycle.
Tamoxifen tablets: 10 mg/dose, oral administration, twice daily, with 28 consecutive days of administration counted as one treatment cycle.

### 1.4 Evaluation indicators and criteria

### Efficacy evaluation indicators:

Invasive disease-free survival (IDFS): the time from the date of randomization to the first occurrence of the following events, including ipsilateral or contralateral recurrent invasive breast cancer, regional or distant recurrent invasive breast cancer, second primary malignancy at a non-breast site, or death from any cause.

Distant relapse free survival (DRFS): the time from the date of randomization to the first occurrence of distant recurrence or death from any cause.

Overall survival (OS): the time from the date of randomization to the date of death from any cause.

### Efficacy evaluation criteria:

The median IDFS and 95% confidence interval (CI) were estimated using the Kaplan-Meier method, and a Kaplan-Meier curve was plotted. Baseline risk is assumed to be different for each stratum. Based on this hypothesis, the comparison between groups was performed using the stratified Log-rank test, and the hazard ratio (HR) between the groups was estimated using the stratified Cox proportional hazards model. The median OS and 95% CI were estimated using the Kaplan-Meier method, and a Kaplan-Meier curve was plotted. The comparison between the groups was performed using the results of the stratified Log-rank test, and the HR between the groups was estimated using the stratified Cox proportional hazards model.

### Safety evaluation indicator:

The incidence and severity of adverse events (AEs), abnormal laboratory test values, and severe adverse events (SAEs).

### Safety evaluation criteria:

The severity of adverse events was determined using the NCI-CTC AE 5.0 criteria.

### 1.5 Study results

### 1.5.1. Safety

The main adverse events were gastrointestinal reactions, and most of the TRAEs were grade 1-2, which could be controlled after symptomatic treatment, and no grade 4-5 TRAEs and treatment-related deaths were observed.

### 1.5.2. Efficacy

The median follow-up time was 3-4 years. The invasive disease-free survival (IDFS), distant relapse free survival (DRFS), and overall survival (OS) of the test group were improved compared with those of the control group. For example, the IDFS could be increased by more than 5%.

### Experimental Example 2

The study involved 2 cohorts, namely cohort I and cohort II, and the study drugs were the compound of formula (I) and an aromatase inhibitor in combination for both cohorts. Subjects with HR-positive and HER2-negative locally advanced and/or metastatic breast cancer were enrolled, with 30-60 subjects enrolled in each cohort. The preliminary efficacy and safety of the compound of formula (I) in combination with the aromatase inhibitor were evaluated. The aromatase inhibitor is selected from the group consisting of letrozole and anastrozole, and the two drugs are each administered to 15-30 subjects per cohort.

### 2.1 Inclusion criteria:

1) Subjects with voluntary participation, signed informed consent, and good compliance;
2) Aged 18-75 years (when signing the informed consent); ECOG PS score: 0-1; expected survival time of more than 3 months;
3) Postmenopausal or premenopausal/perimenopausal female patients;
4) Patients with breast cancer diagnosed as HR-positive and HER2-negative by pathological examination;
5) Subjects enrolled in cohort I relapsed or progressed during an adjuvant endocrine therapy or after completion of an adjuvant endocrine therapy;
6) Completion of an adjuvant endocrine therapy in cohort I was defined as consecutive treatment for at least 2 years and then interruption;
7) Subjects enrolled in cohort II had not previously received any whole-body systemic anti-tumor therapy for local lesion recurrence or metastatic diseases;
8) Having at least one measurable lesion as confirmed according to RECIST 1.1 criteria;
9) With good main organ functions meeting the following criteria:

Blood routine examination criteria (no blood transfusion and no correction using hematopoietic stimulating drugs within the last 7 days before screening):
a) Hemoglobin (HB) ≥ 100 g/L;
b) Absolute value of neutrophil count (NEUT) ≥ 1.5 × 10⁹/L;
c) Platelet count (PLT) ≥ 90 × 10⁹/L.

Biochemical test results should meet the following criteria:
a) Total bilirubin (TBIL) ≤ 2.5 times of upper limit of normal (ULN);
b) Alanine aminotransferase (ALT) and aspartate aminotransferase (AST) ≤ 2.5 × ULN. If liver metastasis is accompanied, ALT and AST ≤ 5 × ULN;
c) Serum creatinine (CR) ≤ 1.5 × ULN or creatinine clearance rate (CCR) ≥ 60 mL/min.

Blood coagulation function examination should meet the following criteria:
Prothrombin time (PT), activated partial thromboplastin time (APTT), and international normalized ratio (INR) ≤ 1.5 × ULN (not previously received anticoagulant therapy).

Heart color ultrasound evaluation: left ventricular ejection fraction (LVEF) ≥ 50%.

10) Female subjects of child-bearing age should agree to take contraceptive measures (such as intrauterine devices, contraceptives, or condoms) during the study and within 6 months after the study; serum pregnancy test results should be negative within 7 days before the study enrollment, and the subjects must not be breastfeeding.

### 2.2 Study drugs

Capsules of the compound of formula (I): strength: 60 mg.
Letrozole tablets: strength: 2.5 mg.
Anastrozole tablets: strength: 1 mg.

### 2.3 Administration regimen

### (1) Compound of formula (I):

Capsules of the compound of formula (I): 180 mg (based on the mass of compound of formula (I)), oral administration at fasting, once daily, with 28 consecutive days of administration counted as one treatment cycle.

(2) Aromatase inhibitor: letrozole or anastrozole may be selected, and the specific administration regimen is as follows:
Letrozole tablets: 2.5 mg (based on the mass of letrozole), oral administration, once daily, with 28 consecutive days of administration counted as one treatment cycle. The tablets were administered together with the capsules of the compound of formula (I).
Anastrozole tablets: 1 mg (based on the mass of anastrozole), oral administration, once daily, with 28 consecutive days of administration counted as one treatment cycle. The tablets were administered together with the capsules of the compound of formula (I).

### 2.4. Evaluation criteria

Efficacy evaluation criteria: disease status was determined using the RECIST 1.1 criteria.
Safety evaluation criteria: the severity of adverse events was determined using the NCI-CTC AE 5.0 criteria.

### 2.5 Study results

### 2.5.1 Safety

A statistical analysis of the safety in 43 subjects showed that the main adverse events were gastrointestinal reactions, and most of the TRAEs were grade 1-2, which could be controlled after symptomatic treatment; the overall incidence of grade 3 TRAEs was only 27.9%; and no grade 4-5 TRAEs and treatment-related deaths were observed.

### 2.5.2 Efficacy

Of the 43 subjects, 38 subjects were enrolled in cohort II and the others were enrolled in cohort I. 35 subjects (cohort II: 32 subjects, cohort I: 3 subjects) were available for efficacy evaluation. Efficacy results showed that the ORR was 65.7% (23/35), wherein the subjects in cohort II had an effective rate of 65.6% (21/32), which was consistent with the ORR of overall population, and the clinical benefit rate (CBR) of cohort II was 90.6% (29/32); the subjects in cohort I had an effective rate of 66.7% (2/3), which was consistent with the ORR of overall population, and the clinical benefit rate (CBR) of cohort I was 100% (3/3). The proportion of 43 subjects with disease-free survival (DFS) of more than 3 years was greater than 75%.

Conclusion: the combined pharmaceutical composition of the present disclosure can exhibit good clinical benefits. The following is an overview of representative subjects:

| Subject No. | Dose group | Cohort | Administration cycle | Efficacy evaluation | | | | | |
|---|---|---|---|---|---|---|---|---|---|
| | | | | C2 | C4 | C6 | C8 | C10 | C12 |
| 1 | Capsules of the compound of formula (I): 180 mg/dose, once daily, with 28 days counted as one treatment cycle; | 2 | C13 | SD (-21.4%*) | PR (-41.6%*) | NA | PR (-48.9%*) | PR (-43.3%*) | PR (-53.7%*) |
| | letrozole: 2.5 mg/dose, once daily, with 28 days counted as one treatment cycle. | | | | | | | | |
| 2 | Same as No. 1 | 2 | C13 | SD (-20%*) | SD (-25%*) | NA | PR (-40%*) | PR (-40%*) | PR (-40%*) |
| 3 | Same as No. 1 | 2 | C13 | PR (-51.9%*) | PR (-51.9%*) | PR (-51.9%*) | PR (-51.9%*) | PR (-51.9%*) | PR (-51.9%*) |
| 4 | Same as No. 1 | 2 | C11 | PR (-63.6%*) | PR (-63.6%*) | PR (-63.6%*) | PR (-63.6%*) | PR (-63.6%*) | -- |
| 5 | Same as No. 1 | 2 | C9 | PR (-31.5%*) | PR (-44.7%*) | PR (-58.8%*) | PR (-62.6%*) | -- | -- |
| 6 | Capsules of the compound of formula (I): 180 mg/dose, once daily, with 28 days counted as one treatment cycle; | 2 | C12 | PR (-31.4%*) | PR (-57.6%*) | NA | PR (-64.9%*) | PR (-64.9%*) | -- |
| | anastrozole: 1 mg/dose, once daily, with 28 days counted as one treatment cycle. | | | | | | | | |
| 7 | Same as No. 6 | 2 | C12 | PR (-54.1%*) | PR (-39.5%*) | PR (-39.5%*) | PR (-39.5%*) | PR (-53.4%*) | -- |
| 8 | Same as No. 6 | 2 | C11 | SD (-12.1%*) | PR (-33.5%*) | PR (-40.6%*) | PR (-60.8%*) | PR (-58.1*) | -- |
| 9 | Same as No. 6 | 2 | C9 | PR (-54.1%*) | PR (-80.7%*) | PR (-73.3%*) | PR (-71.6%*) | -- | -- |
| 10 | Same as No. 6 | 2 | C9 | SD (-27.0%*) | PR (-34.2%*) | PR (-37.8%*) | PR (-50.3%*) | -- | -- |
| 11 | Capsules of the compound of formula (I): 180 mg/dose, once daily, with 28 days counted as one treatment cycle; | 1 | C12 | SD (-27.3%*) | PR (-63.6%*) | PR (-63.6%*) | PR (-63.6%*) | PR(-63.6%*) | -- |
| | letrozole: 2.5 mg/dose, once daily, with 28 days counted as one treatment cycle. | | | | | | | | |
| 12 | Same as No. 11 | 1 | C11 | SD (-29.4%*) | SD (-29.4%*) | PR (-47%*) | NA | NA | -- |

| | | | | | | | | | |
|---|---|---|---|---|---|---|---|---|---|
| Note: "NA" means not evaluated; "*" indicates tumor volume reduction percentage; and "--" indicates that the tumor evaluation point has not been reached. | | | | | | | | | |

Those skilled in the art will recognize that the scope of the present application is not limited to the various embodiments and examples described above. Instead, various modifications, substitutions, or recombinations can be made without departing from the spirit and concept of the present application, all of which fall within the protection scope of the present application.

## Claims

1. A combined pharmaceutical composition for use in treating or preventing breast cancer in a patient who has received surgical treatment, comprising a compound of formula (I) or a pharmaceutically acceptable salt thereof and an endocrine therapeutic agent,

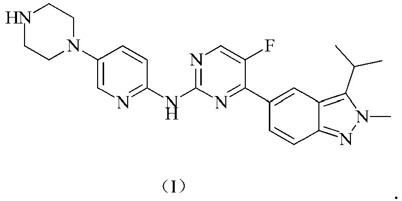

2. The combined pharmaceutical composition for use according to claim 1, wherein the endocrine therapeutic agent is selected from one or two of an aromatase inhibitor, an estrogen receptor antagonist, or a pituitary gonadotropin-releasing hormone analogue.

3. The combined pharmaceutical composition for use according to claim 1 or 2, wherein the endocrine therapeutic agent is selected from the group consisting of an estrogen receptor antagonist, a pituitary gonadotropin-releasing hormone analogue, a combination of an aromatase inhibitor and a pituitary gonadotropin-releasing hormone analogue, and a combination of an estrogen receptor antagonist and a pituitary gonadotropin-releasing hormone analogue.

4. The combined pharmaceutical composition for use according to any one of claims 1-3, wherein the aromatase inhibitor is selected from the group consisting of letrozole and anastrozole;
the estrogen receptor antagonist is selected from tamoxifen; and/or
the pituitary gonadotropin-releasing hormone analogue is selected from goserelin.

5. The combined pharmaceutical composition for use according to any of claims 1-4, wherein the endocrine therapeutic agent is selected from the group consisting of letrozole, anastrozole, tamoxifen, a combination of letrozole and goserelin, a combination of anastrozole and goserelin, and a combination of tamoxifen and goserelin.

6. The combined pharmaceutical composition for use according to any one of claims 1-5, wherein the pharmaceutically acceptable salt of the compound of formula (I) is a maleate, preferably a monomaleate.

7. The combined pharmaceutical composition for use according to any of claims 1-6, wherein the combined pharmaceutical composition comprises the compound of formula (I) or the pharmaceutically acceptable salt thereof and the endocrine therapeutic agent in a mass ratio of 1:1 to 200:1, 10:1 to 200:1, 6:1 to 180:1, 6:1 to 90:1, 6:1 to 9:1, 48:1 to 72:1, 120:1 to 180:1, or any ratio within the range described above, wherein the amount of the compound of formula (I) or the pharmaceutically acceptable salt thereof is based on the mass of the compound of formula (I), and the amount of the endocrine therapeutic agent is based on the mass of an active ingredient therein.

8. The combined pharmaceutical composition according to any one of claims 1-6, wherein the combined pharmaceutical composition comprises 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I), or a pharmaceutical composition thereof.

9. The combined pharmaceutical composition for use according to claim 4, wherein the combined pharmaceutical composition comprises 2.5-12.5 mg, 2.5-10 mg, 2.5-7.5 mg, or 2.5-5 mg of letrozole or a pharmaceutical composition thereof;
the combined pharmaceutical composition comprises 1-5 mg, 1-4 mg, 1-3 mg, or 1-2 mg of anastrozole or a pharmaceutical composition thereof; and/or
the combined pharmaceutical composition comprises 10-40 mg or 20-40 mg of tamoxifen or a pharmaceutical composition thereof.

10. The combined pharmaceutical composition for use according to claim 4, wherein the combined pharmaceutical composition is a formulation suitable for administration in a single treatment cycle (e.g., 28 days), and the formulation comprises: a total amount of 1680-5040 mg of the compound of formula (I) or the pharmaceutically acceptable salt thereof or a pharmaceutical composition thereof, and a total amount of 70-140 mg of letrozole or a pharmaceutical composition thereof, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole; or
the combined pharmaceutical composition is a formulation suitable for administration in a single treatment cycle (e.g., 28 days), and the formulation comprises: a total amount of 1680-5040 mg of the compound of formula (I) or the pharmaceutically acceptable salt thereof or a pharmaceutical composition thereof, and a total amount of 28-140 mg of anastrozole or a pharmaceutical composition thereof, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

11. The combined pharmaceutical composition for use according to claim 4, wherein the combined pharmaceutical composition is a formulation suitable for administration in a single treatment cycle (e.g., 28 days), and the formulation comprises: a total amount of 1680-5040 mg of the compound of formula (I) or the pharmaceutically acceptable salt thereof or a pharmaceutical composition thereof, and a total amount of 560-1120 mg of tamoxifen or a pharmaceutical composition thereof, wherein the amount of the compound of formula (I) or the pharmaceutically acceptable salt thereof or the pharmaceutical composition thereof is based on the mass of the compound of formula (I), and the amount of tamoxifen or the pharmaceutical composition thereof is based on the mass of tamoxifen.

12. The combined pharmaceutical composition for use according to any one of claims 8-11, wherein the pharmaceutical composition of the compound of formula (I) is in a single dose or multiple doses; and/or
the pharmaceutical composition of letrozole or the pharmaceutical composition of anastrozole, and the pharmaceutical composition of tamoxifen are in a single dose or multiple doses.

13. Use of a compound of formula (I) or a pharmaceutically acceptable salt thereof in combination with an endocrine drug for preparing a medicament for treating or preventing breast cancer in a patient who has received surgical treatment,

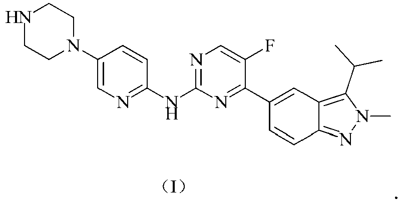

14. A method for treating or preventing breast cancer in a patient who has received surgical treatment, comprising administering to an individual in need thereof a therapeutically effective amount of a compound of formula (I) or a pharmaceutically acceptable salt thereof and an endocrine therapeutic agent.

15. The combined pharmaceutical composition for use according to any one of claims 1-12, the use according to claim 13, or the method according to claim 14, wherein the compound of formula (I) or the pharmaceutically acceptable salt thereof and the endocrine therapeutic agent are used for adjuvant therapy of breast cancer;
the surgical treatment is selected from the group consisting of radical surgery and breast-conserving surgery;
the breast cancer is selected from early-stage breast cancer;
the breast cancer is selected from HR-positive, HER2-negative breast cancer that has received surgical treatment;
the breast cancer is selected from HR-positive, HER2-negative early-stage breast cancer that has received surgical treatment;
the breast cancer is selected from lymph node-positive, HR-positive, HER2-negative breast cancer that has received surgical treatment;
the breast cancer is selected from lymph node-positive, HR-positive, HER2-negative early-stage breast cancer that has received surgical treatment;
the breast cancer is selected from lymph node-positive, HR-positive, HER2-negative breast cancer with a ki-67 index ≥ 20%; or
the breast cancer is selected from lymph node-positive, HR-positive, HER2-negative early-stage breast cancer with a ki-67 index ≥ 20% and a high risk of recurrence.
